# EUROPÄISCHE PATENTSCHRIFT

(11) **EP 2 144 885 B1**
(45) Veröffentlichungstag und Bekanntmachung des Hinweises auf die Patenterteilung: **29.06.2016**
(21) Anmeldenummer: 08734683.9
(22) Anmeldetag: 20.03.2008
(51) Int. Cl.: C07D 233/78, C07D 405/12, C07D 409/12, C07D 471/04, A61K 31/41, A61K 31/435, A61P 3/00

(54) **IMIDAZOLIDIN-CARBONSÄUREAMID-DERIVATE ALS INHIBITOREN VON LIPASEN UND PHOSPHOLIPASEN**
IMIDAZOLIDINE CARBOXAMIDE DERIVATIVES AS LIPASE AND PHOSPHOLIPASE INHIBITORS
DÉRIVÉS D'AMIDE D'ACIDE IMIDAZOLIDINE-CARBOXYLIQUE UTILISÉS COMME INHIBITEURS DE LIPASES ET DE PHOSPHOLIPASES

(30) Priorität: 05.04.2007 EP 07007161
(43) Veröffentlichungstag der Anmeldung: 20.01.2010
(73) Patentinhaber: SANOFI, 75008 Paris (FR)
(72) Erfinder: ZOLLER, Gerhard, 65926 Frankfurt am Main (DE); PETRY, Stefan, 65926 Frankfurt am Main (DE); MÜLLER, Günter, 65926 Frankfurt am Main (DE); TENNAGELS, Norbert, 65926 Frankfurt am Main (DE)
(74) Vertreter: Essler, Frank
(86) Internationale Anmeldenummer: PCT/EP2008/002231
(87) Internationale Veröffentlichungsnummer: WO 2008/122352

(56) Entgegenhaltungen:
- WO-A-2004/093872
- WO-A-2004/094393
- WO-A-2004/094394
- JP-A- 3 227 976

## Beschreibung

Die vorliegende Erfindung betrifft Imidazolidin-carbonsäureamid-derivate der allgemeinen Formel I, deren pharmazeutisch anwendbare Salze und deren Verwendung zur Herstellung eines Arzneimittels.

Es sind bereits neben den in der vorliegenden Anmeldung beschriebenen Imidazolidin-carbonsäureamid-derivaten strukturähnliche Verbindungen im Stand der Technik beschrieben, zum Beispiel von Pirkle et al. in Chirality 4, 302-307 (1992), Phenylaminocarbonyl-hydantoine in der JP 11147878 als Fungizide, sowie die Herbizide aus JP 03227976. Strukturähnliche Verbindungen mit pharmakologischer Wirkung sind in der J. Med. Chem. 1995, 38, 923-933 als NK₁-Rezeptor Antagonisten beschrieben.
Verbindungen mit hemmender Wirkung auf die endotheliale Lipase sind im Stand der Technik beispielsweise in der WO2004/094394, WO2004/094393, WO2004/093872 oder der WO2006/111321 beschrieben.

Aufgabe der vorliegenden Erfindung ist die Bereitstellung von alternativen Verbindungen, die eine Hemmung der endothelialen Lipase bewirken.

Gegenstand der Erfindung sind Imidazolidin-carbonsäureamid-derivate der allgemeinen Formel I 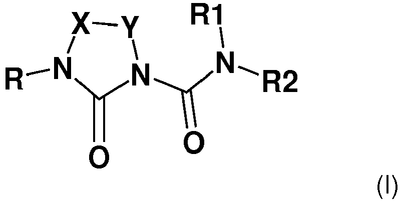 wobei bedeuten:
- X, Y: gleich oder verschieden -C(R3)(R4)-, -(C=O)-, -(C=S), wobei mindestens ein X oder Y für -(C=O)- oder -(C=S)- steht; aber nicht beide gleichzeitig für -(C=O)- oder -(C=S)- stehen können; oder X und Y zusammen für C(R3)=C(R3) stehen;
- R: Wasserstoff, (C₁-C₅)-Haloalkyl, (CR5R6)ₘ-O(R7), (C₁-C₃)-Alkyloxy-(C₁-C₃)-Alkylen, Aryl, Heterocyclus, (C₁-C₄)-Alkylen-Aryl, (C₁-C₄)-Alkylen-Heteroaryl, (C₁-C₄)-Alkylen-(C₅-C₁₂)-Cycloalkyl, wobei Cycloalkyl, Aryl, Heterocyclus oder Heteroaryl durch F, CI, Br, I, OH, CF₃, NO₂, CN, OCF₃, O-(C₁-C₆)-Alkyl, O-(C₁-C₄)-Alkoxy-(C₁-C₄)-Alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, (C₀-C₈)-Alkylen-Aryl, O-(C₀-C₈)-Alkylen-Aryl, S-Aryl, (C₀-C₈)-Alkylen-Heteroaryl, N(R5)(R6), SO₂-CH₃, SO₂-NH₂, SF₅, COOH, COO-(C₁-C₆)-Alkyl, CON(R5)(R6), N(R5)CO(R6), N(R5)SO₂(R6), CO(R5), (CR5R6)_{m'}-(R7), O-CO-N(R5)(R6), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R5)(R6) ein- oder mehrfach substituiert sein kann, wobei Aryl oder Heteroaryl wiederum durch
F, CI, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₂-C₆)-Alkinyl, N(R8)(R9), SO₂-CH₃, SF₅, COOH, COO-(C₁-C₆)-Alkyl, CON(R8)(R9), N(R8)CO(R9), N(R8)SO₂(R9), CO(R8), (CR8R9)_{m"}-O(R10), O-CO-N(R8)(R9), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R8)(R9) ein oder mehrfach substituiert sein kann;
- m, m', m": 0, 1, 2, 3, 4, 5, 6;
- R5, R6, R7, R8, R9, R10: gleich oder verschieden Wasserstoff, (C₁-C₈)-Alkyl;
oder -(C=O)-NR1aR2a;
oder -(C=O)-O-R1b;
oder
- R und X: für X = -C(R3)(R4)- bilden ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem, deren einzelne Glieder der Ringsysteme durch ein bis drei Atome oder Atomgruppen aus der Reihe -CHR11-, -CR11R12-, -(C=R11)-, =C(R11)-, -NR11-, -C(=O)-, -O-, -S-, -SO-, -SO₂-, ersetzt sein können, mit der Maßgabe, dass zwei Einheiten aus der Reihe -O-, -S-, -SO-,-SO₂- nicht benachbart sein dürfen;
- R11, R12: gleich oder verschieden Wasserstoff, (C₁-C₆)-Alkyl, Aryl, (C₃-C₁₂)-Cycloalkyl, (C₁-C₄)-Alkylen-Aryl, (C₁-C₃)-Alkylen-(C₃-C₁₂)-Cycloalkyl; wobei Aryl oder Cycloalkyl durch F, CI, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₂-C₆)-Alkinyl, N(R13)(R14), SO₂-CH₃, SO₂-NH₂, SF₅, COOH, COO-(C₁-C₆)-Alkyl, CON(R13)(R14), N(R13)CO(R14), N(R13)SO₂(R14), CO(R13), (CR13R14)ₙ-O(R15), O-CO-N(R13)(R14), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R13)(R14) substituiert sein kann;

- n: 0, 1, 2, 3, 4, 5, 6;
- R13, R14, R15: gleich oder verschieden Wasserstoff, (C₁-C₈)-Alkyl;
- R1, R1a, R1b: gleich oder verschieden (C₅-C₁₆)-Alkyl, CH₂-Aryl, (C₁-C₂)-Alkylen-Heteroaryl, CH₂-(C₅-C₁₂)-Cycloalkyl, wobei Aryl, Heteroaryl oder Cycloalkyl durch F, CI, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, (C₀-C₈)-Alkylen-Aryl, O-(C₀-C₈)-Alkylen-Aryl, S-Aryl, (C₀-C₈)-Alkylen-Heteroaryl, N(R16)(R17), SO₂-CH₃, SO₂-NH₂, SF₅, COOH, COO-(C₁-C₆)-Alkyl, CON(R16)(R17), N(R16)CO(R17), N(R16)SO₂(R17), CO(R16), (CR16R17)ₒ-O(R18), O-CO-N(R16)(R17), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R16)(R17) ein- oder mehrfach substituiert sein kann, wobei Aryl oder Heteroaryl wiederum durch
F, CI, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₂-C₆)-Alkinyl, N(R19)(R20), SO₂-CH₃, SF₅, COOH, COO-(C₁-C₆)-Alkyl, CON(R19)(R20), N(R19)CO(R20), N(R19)SO₂(R20), CO(R19), (CR19R20)_{o'}-O(R21), O-CO-N(R19)(R20), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R19)(R20) ein oder mehrfach substituiert sein kann;
- o, o': 0, 1, 2, 3, 4, 5, 6;
- R16, R17, R18, R19, R20, R21: gleich oder verschieden Wasserstoff, (C₁-C₈)-Alkyl;
oder ein Rest der Formel la 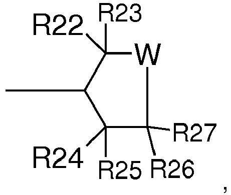 mit
- W: -C(R28)(R29)-, -C(R28)(R29)-C(R28a)(R29a)-, -C(R28)(R29)-O-;
- R22, R23, R24, R25, R26, R27, R28, R29, R28a, R29a: gleich oder verschieden Wasserstoff, F, CI, Br, I, OH, CF₃, NO₂, CN, OCF₃, SF₅, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₃-C₈)-Cycloalkenyl, (C₂-C₆)-Alkinyl, N(R30)(R31), SO₂-CH₃, COOH, COO-(C₁-C₆)-Alkyl, CON(R30)(R31), N(R30)CO(R31), N(R30)SO₂(R31), CO(R30), (CR30R31)ₚ-O(R32), O-CO-N(R30)(R31), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R30)(R31);
- p: 0, 1, 2, 3, 4, 5, 6;
- R30, R31, R32: gleich oder verschieden Wasserstoff, (C₁-C₆)-Alkyl;
oder
R22 und R28 oder R23 und R29 zusammen mit dem sie tragenden Kohlenstoffatomen bilden ein monocyclisches, 5 oder 6 gliederiges gesättigtes, teilweise ungesättigtes oder aromatisches Ringsystem, dessen einzelne Glieder durch -CHR33-, -CR33R34-, =(C-R33)- substituiert sein können;
oder
R24 und R26, oder R25 und R27 zusammen mit dem sie tragenden Kohlenstoffatomen bilden ein monocyclisches, 5 oder 6 gliederiges gesättigtes, teilweise ungesättigtes oder ein aromatisches Ringsystem, dessen einzelne Glieder durch -CHR33-, -CR33R34-, =(C-R33)- substituiert sein können;
- R33, R34: gleich oder verschieden, F, CI, Br, I, OH, CF₃, NO₂, CN, OCF₃, SF₅, O-(C₁-C₆)-Alkyl, O-(C₁-C₄)-Alkoxy-(C₁-C₄)-Alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, N(R35)(R36), SO₂-CH₃, COOH, COO-(C₁-C₆)-Alkyl, CON(R35)(R36), N(R35)CO(R36), N(R35)SO₂(R36), CO(R35), (CR35R36)_{q}-O(R37), O-CO-N(R35)(R36), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R35)(R36);
- q: 0, 1, 2, 3, 4, 5, 6;
- R35, R36, R37: gleich oder verschieden Wasserstoff, (C₁-C₆)-Alkyl;
- R2, R2a: gleich oder verschieden Wasserstoff, (C₁-C₈)-Alkyl;
- R3, R4: gleich oder verschieden Wasserstoff, (C₁-C₆)-Alkyl, Benzyl;
die tautomeren Formen der Verbindungen sowie deren physiologisch verträgliche Salze.

Eine bevorzugte Ausführungsform sind Verbindungen der Formel I, worin R2 Wasserstoff ist.

Bevorzugt sind Verbindungen der Formel I, worin
- X: -(C=O)- und
- Y: -C(R3)(R4)-;
bedeutet.

Bevorzugt sind auch Verbindungen der Formel I, worin
- Y: -(C=O)- und
- X: -C(R3)(R4)-;
bedeutet.

Besonders bevorzugt sind die Verbindungen der Formel I worin
- Y: -(C=O)- und
- X: -C(R3)(R4)-;
oder
- X: -(C=O)- und
- Y: -C(R3(R4)-;
- R: Wasserstoff, (C₁-C₃)-Haloalkyl, (CR5R6)ₘ-0(R7), Phenyl, Heterocyclus, (C₁-C₄)-Alkylen-Phenyl, (C₁-C₄)-Alkylen-Heteroaryl, (C₁-C₄)-Alkylen-(C₅-C₁₂)-Cycloalkyl, wobei Cycloalkyl, Phenyl, Heterocyclus oder Heteroaryl durch F, CI, Br, I, OH, CF₃, NO₂, CN, OCF₃, O-(C₁-C₆)-Alkyl, O-(C₁-C₄)-Alkoxy-(C₁-C₄)-Alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, (C₀-C₈)-Alkylen-Aryl, O-(C₀-C₈)-Alkylen-Aryl, S-Aryl, (C₀-C₈)-Alkylen-Heteroaryl, N(R5)(R6), SO₂-CH₃, SO₂-NH₂, SF₅, COOH, COO-(C₁-C₆)-Alkyl, CON(R5)(R6), N(R5)CO(R6), N(R5)SO₂(R6), CO(R5), (CR5R6)_{m'}-O(R7), O-CO-N(R5)(R6), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R5)(R6) ein- oder mehrfach substituiert sein kann, wobei Aryl oder Heteroaryl wiederum durch
F, CI, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₂-C₆)-Alkinyl, N(R8)(R9), SO₂-CH₃, SF₅, COOH, COO-(C₁-C₆)-Alkyl, CON(R8)(R9), N(R8)CO(R9), N(R8)SO₂(R9), CO(R8), (CR8R9)_{m"}-O(R10), O-CO-N(R8)(R9), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R8)(R9) ein oder mehrfach substituiert sein kann;
- m, m', m": 0, 1, 2, 3, 4, 5, 6;
- R5, R6, R7, R8, R9, R10: gleich oder verschieden Wasserstoff, (C₁-C₈)-Alkyl;
- oder: -(C=O)-NR1aR2a;
- oder: -(C=O)-O-R1b;
oder
- R und X: für X = -C(R3)(R4)- bilden ein monocyclisches, gesättigtes 5- bis 7-gliedriges Ringsystem oder ein bicyclisches teilweise ungesättigtes 8- bis 14 gliedriges Ringsystem, dessen einzelne Glieder durch ein bis drei Atome oder Atomgruppen aus der Reihe -CHR11-, -CR11R12-,-(C=R11)-, -NR11-, -C(=O)-, -O-, ersetzt sein können, mit der Maßgabe, dass zwei Einheiten aus der Reihe -O- nicht benachbart sein dürfen;
- R11, R12: gleich oder verschieden Wasserstoff, (C₁-C₆)-Alkyl, Phenyl, (C₃-C₁₂)-Cycloalkyl, (C₁-C₄)-Alkylen-Phenyl, (C₁-C₃)-Alkylen-(C₃-C₁₂)-Cycloalkyl; wobei Phenyl oder Cycloalkyl durch F, CI, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₂-C₆)-Alkinyl, N(R13)(R14), SO₂-CH₃, SO₂-NH₂, SF₅, COOH, COO-(C₁-C₆)-Alkyl, CON(R13)(R14), N(R13)CO(R14), N(R13)SO₂R14), CO(R13), (CR13R14)ₙ-O(R15), O-CO-N(R13)(R14), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R13)(R14) substituiert sein kann;
- n: 0, 1, 2, 3, 4, 5, 6;
- R13, R14, R15: unabhängig voneinander Wasserstoff, (C₁-C₈)-Alkyl;
- R1, R1a, R1b: gleich oder verschieden (C₅-C₁₂)-Alkyl, -CH₂-Phenyl, (C₁-C₂)-Alkylen-Heteroaryl, -CH₂-(C₅-C₁₂)-Cycloalkyl, (C₅-C₆)-Cycloalkyl, wobei Phenyl, Heteroaryl oder Cycloalkyl durch F, CI, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, (C₀-C₈)-Alkylen-Phenyl, O-(C₀-C₈)-Alkylen-Phenyl, S-Phenyl, (C₀-C₈)-Alkylen-Heteroaryl, N(R16)(R17), SO₂-CH₃, SO₂-NH₂, SF₅, COOH, COO-(C₁-C₆)-Alkyl, CON(R16)(R17), N(R16)CO(R17), N(R16)SO₂(R17), CO(R16), (CR16R17)ₒ-O(R18), O-CO-N(R16)(R17), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R16)(R17) ein- oder mehrfach substituiert sein kann, wobei Phenyl oder Heteroaryl wiederum durch
F, CI, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₂-C₆)-Alkinyl, N(R19)(R20), SO₂-CH₃, SF₅, COOH, COO-(C₁-C₆)-Alkyl, CON(R19)(R20), N(R19)CO(R20), N(R19)SO₂(R20), CO(R19), (CR19R20)_{o'}-O(R21), O-CO-N(R19)(R20), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R19)(R20) ein oder mehrfach substituiert sein kann;
- o, o´: 0, 1, 2, 3, 4, 5, 6;
- R16, R17, R18, R19, R20, R21: gleich oder verschieden Wasserstoff, (C₁-C₈)-Alkyl;
oder ein Rest der Formel Ib 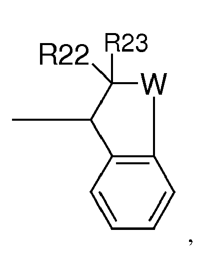 bedeuten.

Ganz besonders bevorzugt sind die Verbindungen der Formel I, worin
- Y: -(C=O)- und
- X: -C(R3)(R4)-;
oder
- X: -(C=O)- und
- Y: -C(R3(R4)-;
- R: Wasserstoff, (CR5R6)ₘ-O(R7), Phenyl, -CH₂-Phenyl, wobei Phenyl durch F, Cl, Br, CF₃, O-(C₁-C₆)-Alkyl, (C₁-C₆)-Alkyl, O-(C₂-C₄)-Haloalkyl, N(R5)(R6), SO₂-CH₃, SO₂-NH₂, COOH, COO-(C₁-C₆)-Alkyl, CON(R5)(R6), CO(R5), (CR5R6)_{m'}-O(R7) ein- oder zweifach substituiert sein kann,
- m, m': 0, 1, 2, 3;
- R5, R6, R7: gleich oder verschieden Wasserstoff, (C₁-C₈)-Alkyl;
oder
- R und X: für X = -C(R3)(R4)- bilden ein monocyclisches, gesättigtes 6-gliedriges Ringsystem oder ein bicyclisches teilweise ungesättigtes 9- bis 11 gliedriges Ringsystem, dessen einzelne Glieder durch ein bis zwei Atome oder Atomgruppen aus der Reihe -CHR11-, -CR11R12-, -(C=R11)-, =C(R11)- ersetzt sein können;
- R11, R12: gleich oder verschieden Wasserstoff, (C₁-C₆)-Alkyl;
- R1: (C₅-C₈)-Alkyl, -CH₂-Phenyl, (C₁-C₂)-Alkylen-Heteroaryl, wobei Heteroaryl ausgewählt ist aus der Gruppe Thiophen, Benzothiophen, Pyridin, Pyrazol, -CH₂-(C₅-C₇)-Cycloalkyl, (C₅-C₆)-Cycloalkyl, wobei Phenyl, Heteroaryl oder Cycloalkyl durch F, CI, Br, OH, CF₃, NO₂, CN, OCF₃, O-(C₁-C₆)-Alkyl, (C₁-C₆)-Alkyl, (C₀-C₈)-Alkylen-Phenyl, O-(C₀-C₈)-Alkylen-Phenyl, (C₀-C₈)-Alkylen-Heteroaryl, N(R16)(R17), SO₂-CH₃, SO₂-NH₂, COOH, COO-(C₁-C₆)-Alkyl, CON(R16)(R17), CO(R16), (CR16R17)ₒ-O(R18) ein- oder mehrfach substituiert sein kann, wobei Phenyl oder Heteroaryl wiederum durch
F, CI, Br, OH, CF₃, NO₂, CN, OCF₃, O-(C₁-C₆)-Alkyl, (C₁-C₆)-Alkyl, COOH, COO-(C₁-C₆)-Alkyl, CON(R19)(R20), CO(R19), (CR19R20)_{o'}-O(R21) ein oder mehrfach substituiert sein kann;
- o, o': 0, 1, 2, 3;
- R16, R17, R18, R19, R20, R21: gleich oder verschieden Wasserstoff, (C₁-C₈)-Alkyl;
oder ein Rest aus der Gruppe 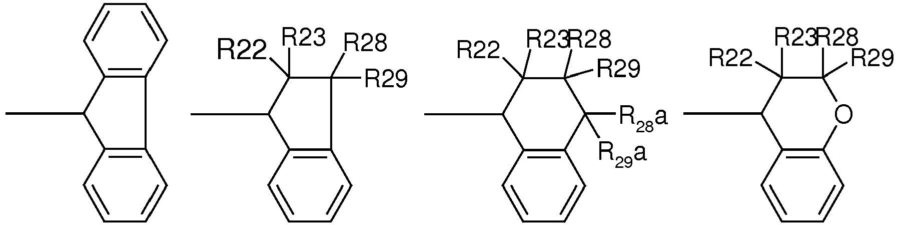 R22, R23, R28, R29, R28a, R29a gleich oder verschieden Wasserstoff, (C₁-C₆)-Alkyl, bevorzugt Wasserstoff und Methyl;
- R2: Wasserstoff;
- R3, R4: gleich oder verschieden Wasserstoff, Methyl;
bedeuten.

Weiterhin besonders bevorzugt sind die Verbindungen der Formel I, worin
- Y: -(C=O)- und
- X: -C(R3)(R4)-;
oder
- X: -(C=O)- und
- Y: -C(R3(R4)-;

- R: Wasserstoff, (CR5R6)ₘ-O(R7), -CH₂-Phenyl, wobei Phenyl durch F, Cl, Br, CF₃, O-(C₁-C₆)-Alkyl, (C₁-C₆)-Alkyl, O-(C₂-C₄)-Haloalkyl, COOH, COO-(C₁-C₆)-Alkyl, CON(R5)(R6), CO(R5), (CR5R6)_{m'}-O(R7) ein- oder zweifach substituiert sein kann,
- m, m': 0, 1, 2, 3;
- R5, R6, R7: gleich oder verschieden Wasserstoff, (C₁-C₄)-Alkyl;
oder
- R und X: für X = -C(R3)(R4)- bilden ein monocyclisches, gesättigtes 6-gliedriges Ringsystem, an welches ein Benzolkern kondensiert sein kann, deren einzelne Glieder der Ringsysteme durch ein bis zwei Atomgruppen aus der Reihe -CHR11-, -CR11R12-, -(C=R11)-, =C(R11)- ersetzt sein können;
- R11, R12: gleich oder verschieden Wasserstoff, (C₁-C₆)-Alkyl;
- R1: (C₅-C₈)-Alkyl, -CH₂-Phenyl, (C₁-C₂)-Alkylen-Heteroaryl, wobei Heteroaryl ausgewählt ist aus der Gruppe Thiophen, Benzothiophen, -CH₂-Cyclohexyl, Cyclohexyl, wobei Phenyl, Heteroaryl oder Cyclohexyl durch F, CI, OH, CF₃, OCF₃, O-(C₁-C₆)-Alkyl, (C₁-C₆)-Alkyl, (C₀-C₁)-Alkylen-Phenyl, O-Phenyl, (C₀-C₁)-Alkylen-Heteroaryl, N(R16)(R17), COOH, COO-(C₁-C₆)-Alkyl, CON(R16)(R17), CO(R16), (CR16R17)ₒ-O(R18) ein-oder zweifach substituiert sein kann, wobei Phenyl oder Heteroaryl wiederum durch
F, CI, OH, CF₃, O-(C₁-C₆)-Alkyl, (C₁-C₆)-Alkyl, COOH, COO-(C₁-C₆)-Alkyl, CON(R19)(R20), CO(R19), (CR19R20)_{o'}-O(R21) ein oder zweifach substituiert sein kann;
- o, o': 0, 1, 2, 3;
- R16, R17, R18, R19, R20, R21: gleich oder verschieden Wasserstoff, (C₁-C₈)-Alkyl;
oder ein Rest aus der Gruppe 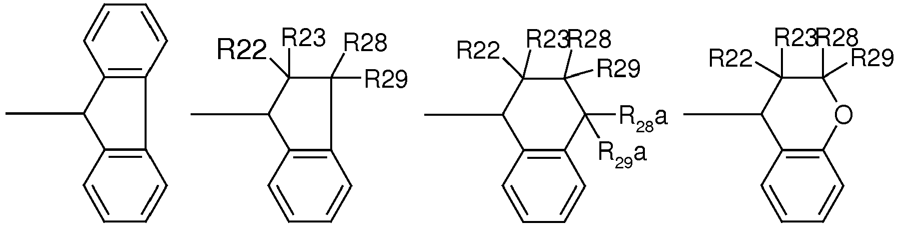 R22, R23, R28, R29, R28a, R29a gleich oder verschieden Wasserstoff, (C₁-C₆)-Alkyl, bevorzugt Wasserstoff und Methyl;
- R2: Wasserstoff;
- R3, R4: gleich oder verschieden Wasserstoff, Methyl;
bedeuten.

Insbesonders besonders bevorzugt sind Verbindungen der Formel I, worin
- Y: -(C=O)- und
- X: -C(R3)(R4)-;
oder
- X: -(C=O)- und
- Y: -C(R3)(R4)-;

- R: Wasserstoff, HO-CH₂-, Benzyl,
oder
- R und X: für X = -C(-R3)(R4)- bilden -CH₂-CH₂-CH₂-CH₂- oder

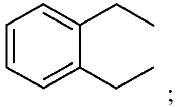

- R1: Pentyl, Hexyl, Heptyl, Cyclohexyl, -CH₂-Cyclohexyl, -CH₂-Phenyl, -CH₂-Thiophen, -CH₂-CH₂-Thiophen, wobei Cyclohexyl, Phenyl oder Thiophen durch Methyl substituiert sein kann;
oder ein Rest aus der Gruppe 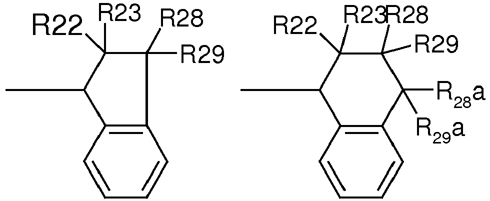
R22, R23, R28, R29, R28a, R29a Wasserstoff;
- R2: Wasserstoff;
- R3, R4: gleich oder verschieden Wasserstoff, Methyl;
bedeuten.

Die Erfindung bezieht sich auf Verbindungen der Formel I, in Form ihrer Salze, Racemate, racemischen Mischungen und reinen Enantiomere sowie auf ihre Diastereomere und Mischungen davon.

Können Reste oder Substituenten mehrfach in den Verbindungen der Formel I auftreten, so können sie alle unabhängig voneinander die angegebenen Bedeutungen haben und gleich oder verschieden sein.

Die Alkyl- oder Alkylenreste in den Substituenten R, R1 bis R37 können sowohl geradkettig wie verzweigt sein. Halogen steht für Fluor, Chlor, Brom oder lod, besonders für Fluor oder Chlor.
Haloalkyl ist ein durch Halogen ein, mehrfach oder vollständig substituiertes Alkyl. Bevorzugte Halogene sind Fluor und Chlor.

Unter einem Cycloalkylrest wird ein einen oder mehrere Ringe enthaltendes Ringssystem, welches gesättigt vorliegt, verstanden, das ausschließlich aus Kohlenstoffatomen aufgebaut ist, wie z.B. Cyclopropyl, Cyclopentyl, Cyclohexyl oder Adamantyl.

Unter einem Arylrest wird ein Phenyl- oder Naphthylrest verstanden.
Die Arylreste können ein oder mehrfach mit geeigneten Gruppen substituiert sein, wie z.B.: F, Cl, Br, I, CF₃, NO₂, CN, COOH, COO(C₁-C₆)Alkyl, CONH₂, CONH(C₁-C₆)Alkyl, CON[(C₁-C₆)Alkyl]_{2,}, (C₃-C₁₀)-Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl,
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl -CO-Aryl, N(C₁-C₆)-Alkyl -CO-Heterocyclus, N(C₁-C₆)-Alkyl -COO-Aryl, N(C₁-C₆)-Alkyl -COO-Heterocyclus, N(C₁-C₆)-Alkyl-CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, CI, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Heterocyclus ist ein mono- oder bicyclisches Ringsystem mit 5 bis 12 Ringgliedern, worin mindestens ein Atom im Ringsystem ein Heteroatom aus der Reihe N, O und S ist. Ferner gehören auch Ringsysteme zu dieser Definition, worin der Heterocyclus mit einem Benzolkern kondensiert ist. (C₅-C₇)-Heterocyclus ist ein monozyklisches, (C₈-C₁₂)-Heterocyclus ein bicyclisches Ringsystem.

Geeignete "Heterocyclische Ringe" bzw. "Heterocyclische Reste" sind Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-lndolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Die Heterocyclischen Ringe bzw. Heterocyclische Reste können ein oder mehrfach mit geeigneten Gruppen substituiert sein, wie z.B.: F, CI, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, CI, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, CI, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Heteroaryl ist ein mono- oder bicyclisches aromatisches Ringsystem mit 5 bis 12 Ringgliedern, worin mindestens ein Atom im Ringsystem ein Heteroatom aus der Reihe N, O und S ist. Ferner gehören auch Ringsysteme zu dieser Definition, worin der Heteroaryl mit einem Benzolkern kondensiert ist.
Geeignete "Heteroaryl-Ringe" bzw. "Heteroaryl-Reste" sind beispielsweise Benzimidazolyl, Benzofuranyl, Benzothiophenyl, Benzoxazolyl, Benzthiazolyl, Benztriazolyl, Benztetrazolyl, Benzisoxazolyl, Benzisothiazolyl, Chinolinyl, Furyl, Furazanyl, Imidazolyl, 1H-Indazolyl, Indolyl, 1,2,3-Oxadiazolyl, 1,2,4-Oxadiazolyl, 1,2,5-Oxadiazolyl, 1,3,4-Oxadiazolyl, Pyrimidinyl, Pyrazinyl, Pyrazolyl, Pyridyl, Pyrrolyl, Thiazolyl, 1,2,3-Thiadiazolyl, 1,2,4-Thiadiazolyl, 1,2,5-Thiadiazolyl, 1,3,4-Thiadiazolyl, Thiophenyl.
Die Heteroaryl-Ringe bzw. Heteroaryl-Reste können ein oder mehrfach mit geeigneten Gruppen substituiert sein, wie z.B.: F, CI, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, CI, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, CI, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure.

Geeignete pharmazeutisch verträgliche basische Salze sind Ammoniumsalze, Alkalimetallsalze (wie Natrium- und Kaliumsalze) und Erdalkalisalze (wie Magnesium-und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I "auf Verbindung(en) der Formel I, wie vorstehend beschrieben, sowie ihre Salze, Solvate und physiologisch funktionelle Derivate wie hierin beschrieben.

### Verwendung

Die Verbindungen der allgemeinen Formel I
besitzen eine überraschende hemmende Wirkung auf die endotheliale Lipase (EL). Das antiatheroskleotisch wirksame HDL ist das bevorzugte Substrat für EL. Eine Senkung des HDL-Spiegels führt zur Progression von Atherosklerose und ihrer Folgeerkrankungen wie Koronare Herzerkrankungen und begünstigt darüberhinaus die Entstehung des Metabolischen Syndroms und seiner Folgeerkrankung Diabetes. Eine Hemmung der EL sollte somit generell zur Vorbeugung von atherosklerotischen Erkrankungen führen und indirekt bei Personen mit erhöhtem Risiko für Diabetes die Erkrankungswahrscheinlichkeit verringern.
Weiterhin wurde gefunden, dass die hemmende Wirkung der erfindungsgemäßen Verbindungen der allgemeinen Formel I selektiv gegenüber anderen Lipasen ist.

Die Verbindungen der Formel I zeigen eine außerdem eine verbesserte Löslichkeit in wässrigen Medien bei mindestens gleich hoher Aktivität, verglichen mit Verbindungen ähnlicher Strukturen. Die erfindungsgemäßen Verbindungen zeichnen sich weiterhin durch weitere vorteilhafte Eigenschaften, wie höhere metabolische Stabilität und Serumstabilität gegenüber Verbindungen des Standes der Technik aus.

Besonders geeignet sind solche Verbindungen zur Behandlung und/oder Prävention von
1. Dyslipidämien und allgemeine Störungen des Fettstoffwechsels und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
2. Verschiedenen andere Zuständen, die mit dem Metabolischen Syndrom assoziert sein können, sind wie:
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
   - Diabetes mellitus, insbesondere Typ 2 Diabetes einschließlich der Verhinderung der damit verbundenen Folgeerkrankungen (Hyperglykämie, Glucoseintoleranz, Verlust der ß-Zellen der Bauchspeicheldrüse, makro- und mikrovaskulärer Erkrankungen
3. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzinome, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und harnableitenden Organe, des Genitaltraktes, Prostata-Karzinome etc.
   - akute und chronische myeloprolifeative Erkrankungen und Lymphome
   - Angiogenese
   - Neurodegenerative Erkrankungen
   - Alzheimersche Krankheit
   - Multiple Sklerose
   - Morbus Parkinson
   - Erythemato-squamöse Dermatosen, wie z.B. Psoriasis (Schuppenflechte)
   - Akne vulgaris
   - Andere Hautkrankheiten und dermatologische Zustände, die durch PPAR moduliert werden
   - Ekzeme und Neurodermitis
   - Dermatitiden, wie z.B. seborrhoische Dermatitis oder Lichtdermatitis
   - Keratitis und Keratosen, wie z.B. seborrhoische Keratosen, senile Keratosen, aktinische Keratose, photo-induzierte Keratosen oder Keratosis follicularis
   - Keloide und Keloid-Prophylaxe
   - Warzen, einschließlich Kondylomata oder Kondylomata acuminata
   - Human papilloma viral (HPV) Infektionen, wie z.B. venerische Papillomata, virale Warzen, wie z.B. Molluscum contagiosum, Leukoplakie
   - Papulöse Dermatosen, wie z.B. Lichen planus
   - Hautkrebs, wie z.B. Basalzellkarzinome, Melanome oder kutane T-Zell Lymphome
   - Lokalisierte, benigne epidermale Tumore, wie z.B. Keratoderma, epidermale Naevi
   - Frostbeulen
   - Hoher Blutdruck
   - Syndrom X
   - Syndrom der polyzystischen Ovarien (PCOS)
   - Asthma
   - Osteoarthritis
   - Lupus erythematodes (LE) oder entzündliche rheumatische Erkrankungen, wie z.B. Rheumatoide Arthritis
   - Vaskulitis
   - Auszehrung (Kachexie)
   - Gicht
   - Ischämie/Reperfusions Syndrom
   - Akutes respiratorisches Distress Syndrom (ARDS) ("Schocklunge")

### Galenik

Die Menge einer erfindungsgemäßen Verbindung, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3 -10 mg/kg/Tag. Eine intravenöse Dosis kann z.B. im Bereich von 0,3 mg bis 1,0 mg/kg liegen, die geeigneter weise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischer weise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer erfindungsgemäßer Verbindungen. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, welche die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneter weise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formel I zeichnen sich durch günstige Wirkungen auf Lipidstoffwechselstörungen aus. Sie beeinflussen das Verhältnis von HDL zu LDL positiv und erhöhen besonders die HDL-Spiegel und sind zur Prävention und Behandlung von Dyslipidämien und Metabolischem Syndrom sowie deren vielfältigen Folgeerkrankungen wie Atherosklerose, koronare Herzerkrankungen, Herzinsuffizienz, Adipositas und Diabetes geeignet.

### Kombinationen mit anderen Medikamenten

Die erfindungsgemäßen Verbindungen können alleine oder in Kombination mit einem oder mehreren weiteren pharmakologischen Wirkstoffen verabreicht werden. Insbesondere können die erfindungsgemäßen Verbindungen mit Wirkstoffen, die eine ähnliche pharmakologische Wirkung wie sie selbst aufweisen, verabreicht werden. Beispielsweise können sie in Kombination mit Wirkstoffen, die eine günstige Wirkungen auf Stoffwechselstörungen bzw. häufig damit assoziierte Erkrankungen haben, verabreicht werden. Beispiele für solche Medikamente sind
1. blutzuckersenkende Medikamente, Antidiabetika,
2. Wirkstoffe zur Behandlung von Dyslipidämien,
3. antiatherosklerotische Medikamente,
4. Mittel gegen Obesitas,
5. entzündungshemmende Wirkstoffe
6. Wirkstoffe zur Behandlung von malignen Tumoren
7. antithrombotische Wirkstoffe
8. Wirkstoffe zur Behandlung von Bluthochdruck
9. Wirkstoffe zur Behandlung von Herzinsuffizienz und
10. Wirkstoffe zur Behandlung und/oder Prävention von durch Diabetes verursachten bzw. mit Diabetes assoziierten Komplikationen.
11. Wirkstoffe zur Behandlung von neurodegenerativen Krankheiten
12. Wirkstoffe zur Behandlung von Krankheiten des Zentralen Nervensystems
13. Wirkstoffe zur Behandlung von Drogen-, Nikotin- und Alkoholabhängigkeit
14. Schmerzmittel

Sie lassen sich mit den erfindungsgemäßen Verbindungen der Formel I insbesondere zur synergistischen Wirkungsverbesserung kombinieren. Die Verabreichung der Wirkstoffkombination kann entweder durch getrennte Verabreichung der Wirkstoffe an den Patienten oder in Form von Kombinationsprodukten, bei denen mehrere Wirkstoffe in einer pharmazeutischen Zubereitung vorhanden sind, erfolgen.

Als weitere Wirkstoffe für die Kombinationspräparate sind insbesondere geeignet: Alle Antidiabetika, die in der Roten Liste 2006, Kapitel 12 genannt sind; alle Abmagerungsmittel/Appetitzügler, die in der Roten Liste 2006, Kapitel 1 genannt sind; alle Lipidsenker, die in der Roten Liste 2006, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964 oder solche, wie sie in WO2005005477 (Novo Nordisk) beschrieben sind, schnell wirkende Insuline (siehe US 6,221,633), inhalierbare Insuline, wie z. B. Exubera^{®} oder orale Insuline, wie z. B. IN-105 (Nobex) oder Oral-lyn™ (Generex Biotechnology), GLP-1-Derivate wie z.B. Exenatide, Liraglutide oder diejenigen die in WO 98/08871 oder WO2005027978 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die Wirkstoffe umfassen vorzugsweise
Sulfonylharnstoffe,
Biguanide,
Meglitinide,
Oxadiazolidindione,
Thiazolidindione,
Glukosidase-Inhibitoren,
Hemmstoffe der Glykogen Phosphorylase,
Glukagon-Antagonisten,
Glukokinase Aktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT), GLP-1-Agonisten,
Kaliumkanalöffner, wie z.B. diejenigen, die in WO 97/26265 und WO 99/03861 von Novo Nordisk A/S offenbart wurden,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption,
Hemmstoffe der 11 ß-HSD1,
Inhibitoren der Protein-Tyrosin-Phosphatase-1B (PTP1 B),
Modulatoren des natrium-abhängigen Glukosetransporters 1 oder 2 (SGLT1, SGLT2), den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindung der Formel I in Kombination mit einem HMGCoA-Reduktase Inhibitor, wie Simvastatin, Fluvastatin, Pravastatin, Lovastatin, Atorvastatin, Cerivastatin, Rosuvastatin oder L-659699, verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692), MD-0727 (Microbia Inc., WO2005021497) oder mit Verbindungen, wie in WO2002066464 (Kotobuki Pharmaceutical Co. Ltd.), WO2005062824 (Merck & Co.) oder WO2005061451 und WO2005061452 (AstraZeneca AB) beschrieben, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483 oder CS-011 (Rivoglitazon), verabreicht.

Bei einer Ausführungsform der Erfindung wird die Verbindung der Formel I in Kombination mit einem PPAR alpha Agonisten, wie z.B. GW9578, GW-590735, K-111, LY-674, KRP-101 oder DRF-10945, verabreicht.

Bei einer Ausführungsform der Erfindung wird die Verbindung der Formel I in Kombination mit einem gemischten PPAR alpha/gamma Agonisten, wie z.B. Muraglitazar, Tesaglitazar, Naveglitazar, LY-510929, ONO-5129, E-3030 oder wie in PCT/US 00/11833, PCT/US 00/11490, DE10142734.4 oder in J.P. Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516, verabreicht.

Bei einer Ausführungsform der Erfindung wird die Verbindung der Formel I in Kombination mit Metaglidasen oder mit MBX-2044 oder anderen partiellen PPAR gamma Agonisten/Antagonisten verabreicht.

Bei einer Ausführungsform der Erfindung wird die Verbindung der Formel I in Kombination mit einem Fibrat, wie z.B. Fenofibrat, Clofibrat oder Bezafibrat, verabreicht.

Bei einer Ausführungsform der Erfindung wird die Verbindung der Formel I in Kombination mit einem MTP-Inhibitor, wie z.B. Implitapide, BMS-201038, R-103757 oder solchen wie in WO2005085226 beschrieben, verabreicht.

Bei einer Ausführungsform der Erfindung wird die Verbindung der Formel I in Kombination mit einem CETP-Inhibitor, wie z.B. Torcetrapib oder JTT-705, verabreicht.

Bei einer Ausführungsform der Erfindung wird die Verbindung der Formel I in Kombination mit einem Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin oder Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586 oder solchen wie in WO2005097738 beschrieben, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, ß-Caroten oder Selen, verabreicht.

Bei einer Ausführungsform der Erfindung wird die Verbindung der Formel I in Kombination mit einem Vitamin, wie z. B. Vitamin B6 oder Vitamin B12, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform der Erfindung wird die Verbindung der Formel I in Kombination mit einem Squalen Synthetase Inhibitor, wie z.B. BMS-188494 oder wie in WO2005077907 beschrieben, verabreicht.

Bei einer Ausführungsform der Erfindung wird die Verbindung der Formel I in Kombination mit einem Lipoprotein(a) Antagonist, wie z.B. Gemcabene (Cl-1027), verabreicht.

Bei einer Ausführungsform der Erfindung wird die Verbindung der Formel I in Kombination mit einem HM74A Rezeptor Agonisten, wie z.B. Nicotinsäure, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform der Erfindung wird die Verbindung der Formel I in Kombination mit einem Sulfonylharnstoff, wie z.B. Tolbutamid, Glibenclamid, Glipizid oder Glimepirid, verabreicht.

Bei einer Ausführungsform der Erfindung wird die Verbindung der Formel I in Kombination mit einem Biguanid, wie z.B. Metformin, verabreicht.

Bei wieder einer Ausführungsform der Erfindung wird die Verbindung der Formel I in Kombination mit einem Meglitinid, wie z.B. Repaglinide oder Nateglinid, verabreicht.

Bei einer Ausführungsform der Erfindung wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform der Erfindung wird die Verbindung der Formel I in Kombination mit einem α-Glukosidase-Inhibitor, wie z.B. Miglitol oder Acarbose, verabreicht.

Bei einer Ausführungsform der Erfindung wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform der Erfindung wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc., verabreicht.

Bei einer Ausführungsform der Erfindung wird die Verbindung der Formel I in Kombination mit einem Hemmstoff der Glykogen Phosphorylase, wie z.B. PSN-357 oder FR-258900 oder solchen wie in WO2003084922, WO2004007455, WO2005073229-31 oder WO2005067932 beschrieben, verabreicht.

Bei einer Ausführungsform der Erfindung wird die Verbindung der Formel I in Kombination mit Glukagon-Rezeptor-Antagonisten, wie z.B. A-770077, NNC-25-2504 oder wie in WO2004100875 oder WO2005065680 beschrieben, verabreicht.

Bei einer Ausführungsform der Erfindung wird die Verbindung der Formel I in Kombination mit Aktivatoren der Glukokinase, wie z. B. RO-4389620, LY-2121260 (WO2004063179), PSN-105, PSN-110, GKA-50oder solchen wie sie z. B. von Prosidion in WO2004072031, WO2004072066, WO 05103021 oder WO 06016178, von Roche in WO 00058293, WO 00183465, WO 00183478, WO 00185706, WO 00185707, WO 01044216, GB 02385328, WO 02008209, WO 02014312, WO 0246173, WO 0248106, DE 10259786, WO 03095438, US 04067939 oder WO 04052869, von Novo Nordisk in EP 1532980, WO 03055482, WO 04002481, WO 05049019, WO 05066145 oder WO 05123132, von Merck/Banyu in WO 03080585, WO03097824, WO 04081001, WO 05063738 oder WO 05090332, von Eli Lilly in WO 04063194, oder von Astra Zeneca in WO 01020327, WO 03000262, WO 03000267, WO 03015774, WO 04045614, WO 04046139, WO 05044801, WO 05054200, WO 05054233, WO 05056530, WO 05080359, WO 05080360 oder WO 05121110 beschrieben sind, verabreicht.

Bei einer Ausführungsform der Erfindung wird die Verbindung der Formel I in Kombination mit einem Inhibitor der Glukoneogenese, wie z. B. FR-225654, verabreicht.

Bei einer Ausführungsform der Erfindung wird die Verbindung der Formel I in Kombination mit Inhibitoren der Fructose-1,6-bisphosphatase (FBPase), wie z.B. CS-917, verabreicht.

Bei einer Ausführungsform der Erfindung wird die Verbindung der Formel I in Kombination mit Modulatoren des Glukosetransporters-4 (GLUT4), wie z.B. KST-48 (D.-O. Lee et al.: Arzneim.-Forsch. Drug Res. 54 (12), 835 (2004)), verabreicht.

Bei einer Ausführungsform der Erfindung wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform der Erfindung wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X oder wie sie in WO2003074500, WO2003106456, WO200450658, WO2005058901, WO2005012312, WO2005/012308, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform der Erfindung wird die Verbindung der Formel I in Kombination mit Hemmstoffen der 11-beta-Hydroxysteroid-Dehydrogenase-1 (11 β-HSD1), wie z. B. BVT-2733 oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004065351, WO2004089367, WO2004089380, WO2004089470-71, WO2004089896, WO2005016877 oder WO2005097759 beschrieben sind, verabreicht.

Bei einer Ausführungsform der Erfindung wird die Verbindung der Formel I in Kombination mit Inhibitoren der Protein-Tyrosin-Phosphatase-1 B (PTP1 B), wie sie z. B. in WO200119830-31, WO200117516, WO2004506446, WO2005012295, PCT/EP2005/005311, PCT/EP2005/005321, PCT/EP2005/007151, PCT/EP2005/ oder DE 10 2004 060542.4 beschrieben sind, verabreicht.

Bei einer Ausführungsform der Erfindung wird die Verbindung der Formel I in Kombination mit Modulatoren des natrium-abhängigen Glukosetransporters 1 oder 2 (SGLT1, SGLT2), wie z.B. KGA-2727, T-1095 und SGL-0010 oder wie sie z. B. in WO2004007517, WO200452903, WO200452902, WO2005121161, WO2005085237, JP2004359630 oder von A. L. Handlon in Expert Opin. Ther. Patents (2005) 15(11), 1531-1540 beschrieben sind, verabreicht.

Bei einer Ausführungsform der Erfindung wird die Verbindung der Formel I in Kombination mit Inhibitoren der hormon-sensitiven Lipase (HSL), wie z. B. in WO2005073199, WO2006111321, WO2006131233, WO2006131232, WO2006131231, WO2007042178, WO2007045392 beschrieben, verabreicht.

Bei einer Ausführungsform der Erfindung wird die Verbindung der Formel I in Kombination mit Hemmstoffen der Acetyl-CoA Carboxylase (ACC) wie z. B. solchen wie in WO199946262, WO200372197, WO2003072197 oder WO2005044814 beschrieben, verabreicht.

Bei einer Ausführungsform der Erfindung wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform der Erfindung wird die Verbindung der Formel I in Kombination mit einem Inhibitor der Glykogen Synthase Kinase-3 beta (GSK-3 beta), wie z. B. in US2005222220, WO2005085230, PCT/EP2005/005346, WO2003078403, WO2004022544, WO2003106410, WO2005058908, US2005038023, WO2005009997, US2005026984, WO2005000836, WO2004106343, EP1460075, WO2004014910, WO2003076442, WO2005087727 oder WO2004046117 beschrieben, verabreicht.

Bei einer Ausführungsform der Erfindung wird die Verbindung der Formel I in Kombination mit einem Inhibitor der Protein Kinase C beta (PKC beta), wie z. B. Ruboxistaurin, verabreicht.

Bei einer Ausführungsform der Erfindung wird die Verbindung der Formel I in Kombination mit einem Endothelin-A-Rezeptor Antagonisten, wie z. B. Avosentan (SPP-301), verabreicht.

Bei einer Ausführungsform der Erfindung wird die Verbindung der Formel I in Kombination mit Inhibitoren der "I-kappaB Kinase" (IKK Inhibitoren), wie sie z. B. in WO2001000610, WO2001030774, WO2004022553 oder WO2005097129 beschrieben sind, verabreicht.

Bei einer Ausführungsform der Erfindung wird die Verbindung der Formel I in Kombination mit Modulatoren des Glukocorticoidrezeptors, wie sie z. B. in WO2005090336 beschrieben sind, verabreicht.

Bei einer weiteren Ausführungsform der Erfindung wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558); NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
Peptid YY 3-36 (PYY3-36) oder analoge Verbindungen, wie z. B. CJC-1682 (PYY3-36 konjugiert mit humanem Serum Albumin über Cys34), CJC-1643 (Derivat des PYY3-36, welches sich in vivo an Serum Albumin konjugiert) oder solche, wie sie in WO2005080424 beschrieben sind;
Cannabinoid Rezeptor 1 Antagonisten, wie z.B. Rimonabant, SR147778 oder solche wie sie in z. B. EP 0656354, WO 00/15609, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509 oder WO2005077897 beschrieben sind;
MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chlorophenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, EP1538159, WO2004072076 oder WO2004072077 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403 oder WO2005075458 beschrieben sind);
Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893 beschrieben sind); CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
β3-Agonisten (wie z.B. 1-(4-Chloro-3-methanesulfonylmethyl-phenyl)-2-[2-(2,3-dimethyl-1 H-indol-6-yloxy)-ethylamino]-ethanol Hydrochlorid (WO 01/83451));
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, WO2004012648, WO2003015769, WO2004072025, WO2005070898, WO2005070925, WO2004039780, WO2003033476, WO2002006245, WO2002002744, WO2003004027 oder FR2868780 beschrieben sind);
CCK-A Agonisten (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525), SR-146131 (WO 0244150) oder SSR-125180);
Serotonin-Wiederaufnahme-Inhibitoren (z.B. Dexfenfluramine);
gemischte Serotonin- und noradrenerge Verbindungen (z.B. WO 00/71549); 5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. APD-356, BVT-933 oder solche, wie sie in WO200077010, WO20077001-02, WO2005019180, WO2003064423, WO200242304 oder WO2005082859 beschrieben sind);
5-HT6 Rezeptor Antagonisten, wie sie z.B. in WO2005058858 beschrieben sind;
Bombesin-Rezeptor Agonisten (BRS-3 Agonisten);
Galanin-Rezeptor Antagonisten;
Wachstumshormon (z.B. humanes Wachstumshormon oder AOD-9604); Wachstumshormon freisetzende Verbindungen (6-Benzyloxy-1-(2-diisopropylamino-ethylcarbamoyl)-3,4-dihydro-1 H-isochinolin-2-carbonsäuretertiärbutylester (WO 01/85695));
Growth Hormone Secretagogue Receptor Antagonisten (Ghrelin Antagonisten) wie z. B. A-778193 oder solche, wie sie in WO2005030734 beschrieben sind; TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin oder Doprexin);
Lipase/Amylase-Inhibitoren (wie sie z.B. in WO 00/40569 beschrieben sind); Inhibitoren der Diacylglycerol O-Acyltransferasen (DGATs) wie z. B. in US2004/0224997, WO2004094618, WO200058491, WO2005044250, WO2005072740, JP2005206492 oder WO2005013907 beschrieben;
Inhibitoren der Fettsäuresynthase (FAS) wie z.B. C75 oder solche, wie in WO2004005277 beschrieben;
Oxyntomodulin;
Oleoyl-Estron
oder Agonisten des Schilddrüsenhormonrezeptors (thyroid hormone receptor agonists) wie z. B: KB-2115 oder solche, wie in WO20058279, WO200172692, WO200194293, WO2003084915, WO2004018421 oder WO2005092316 beschrieben, verabreicht.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform der Erfindung ist der weitere Wirkstoff Dexamphetamin oder Amphetamin.

Bei einer Ausführungsform der Erfindung ist der weitere Wirkstoff Fenfluramin oder Dexfenfluramin.

Bei noch einer Ausführungsform der Erfindung ist der weitere Wirkstoff Sibutramin.

Bei einer Ausführungsform der Erfindung ist der weitere Wirkstoff Mazindol oder Phentermin.

Bei einer Ausführungsform der Erfindung wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6). Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung wird die Verbindung der Formel I in Kombination mit PDE Inhibitoren (Phosphodiesterase), wie sie z. B. in WO2003/077949 oder WO2005012485 beschrieben sind, verabreicht.

Bei einer Ausführungsform der Erfindung wird die Verbindung der Formel I in Kombination mit NAR-1 (Nicotinic Acid Receptor) Agonisten, wie sie z. B. in WO2004094429 beschrieben sind, verabreicht.

Bei einer Ausführungsform der Erfindung wird die Verbindung der Formel I in Kombination mit CB2 (Cannabinoid Receptor) Agonisten, wie sie z. B. in US2005/143448 beschrieben sind, verabreicht.

Bei einer Ausführungsform der Erfindung wird die Verbindung der Formel I in Kombination mit Histamine-1-Agonisten, wie sie z. B. in WO2005101979 beschrieben sind, verabreicht.

Bei einer Ausführungsform der Erfindung wird die Verbindung der Formel I in Kombination mit Bupropion, wie in WO2006017504 beschrieben, verabreicht.

Bei einer Ausführungsform der Erfindung wird die Verbindung der Formel I in Kombination mit Opioid Antagonisten, wie sie z. B. in WO2005107806 oder WO2004094429 beschrieben sind, verabreicht.

Bei einer Ausführungsform der Erfindung wird die Verbindung der Formel I in Kombination mit Neutralen Endopeptidase Inhibitoren , wie sie z. B. in WO200202513, WO2002/06492, WO 2002040008, WO2002040022 oder WO2002047670 beschrieben sind, verabreicht.

Bei einer Ausführungsform der Erfindung wird die Verbindung der Formel I in Kombination mit NPY Inhibitoren (Neuropeptid Y), wie sie z. B. in WO2002047670 beschrieben sind, verabreicht.

Bei einer Ausführungsform der Erfindung wird die Verbindung der Formel I in Kombination mit Natrium/Wasserstoff Austausch Inhibitoren, wie sie z. B. in WO2003092694 beschrieben sind, verabreicht.

Bei einer Ausführungsform der Erfindung wird die Verbindung der Formel I in Kombination mit Modulatoren des Glukocorticoidrezeptors, wie sie z. B. in WO2005090336 beschrieben sind, verabreicht.

Bei einer Ausführungsform der Erfindung wird die Verbindung der Formel I in Kombination mit Nikotin Rezeptor Agonisten, wie sie z. B. in WO2004094429 beschrieben sind, verabreicht.

Bei einer Ausführungsform der Erfindung wird die Verbindung der Formel I in Kombination mit NRIs (Norepinephrine reuptake inhibitors), wie sie z. B. in WO2002053140 beschrieben sind, verabreicht.

Bei einer Ausführungsform der Erfindung wird die Verbindung der Formel I in Kombination mit MOA (E-beta-methoxyacrylate), wie z.B. Segeline oder wie sie z. B. in WO2002053140 beschrieben sind, verabreicht.

Bei einer Ausführungsform der Erfindung wird die Verbindung der Formel I in Kombination mit antithrombotischen Wirkstoffen, wie z. B. Clopidrogel, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Zu den vorstehend genannten Entwicklungskodes werden nachfolgend teilweise die Formeln aufgeführt.

Eine weitere Ausführungsform der Erfindung ist ein Arzneimittel gemäß Anspruch 8, dadurch gekennzeichnet, dass es eine Verbindung der Formel I, wie in Anspruch 8 beschrieben enthält, sowie einen weiteren Wirkstoff, wie in Anspruch 8 beschrieben enthält.

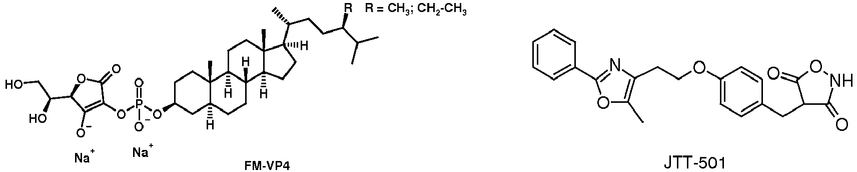

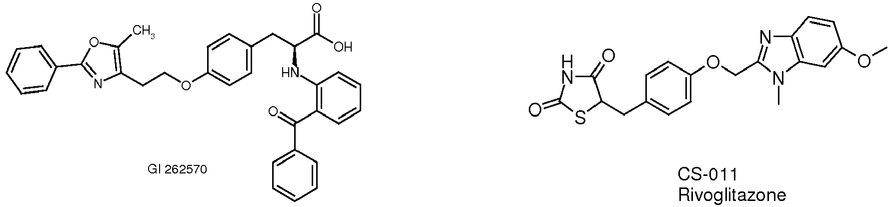

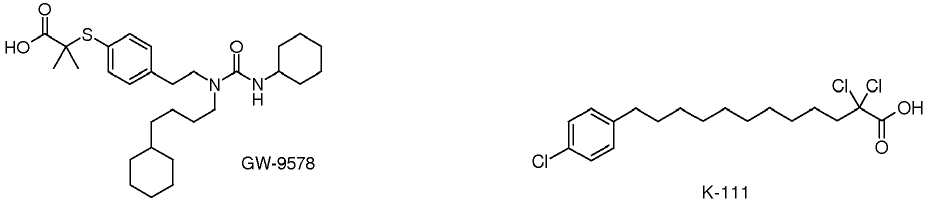

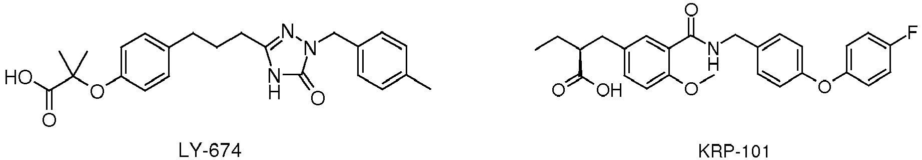

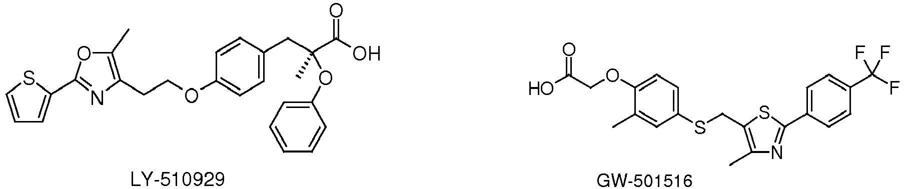

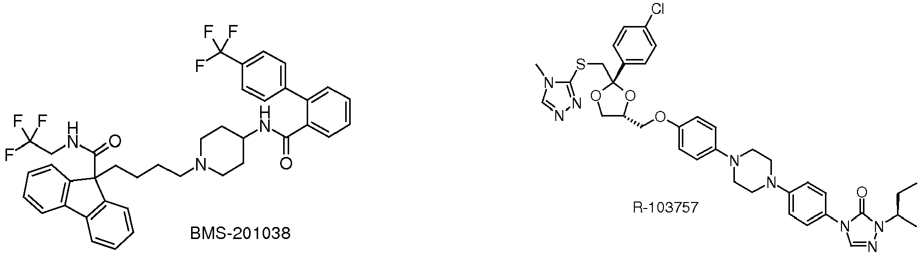

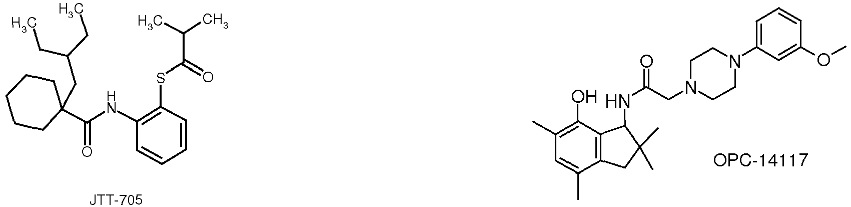

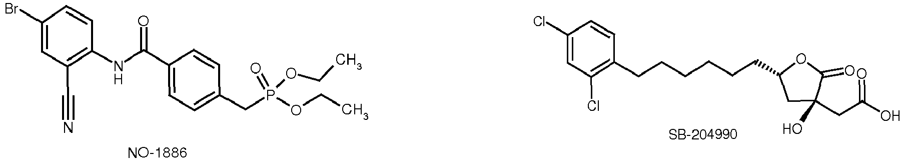

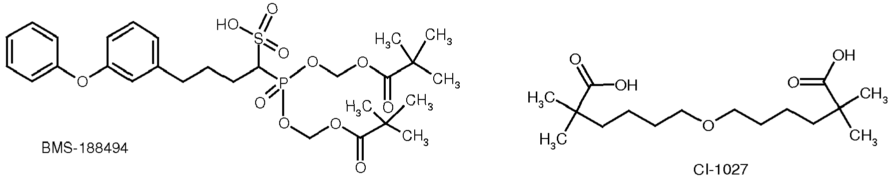

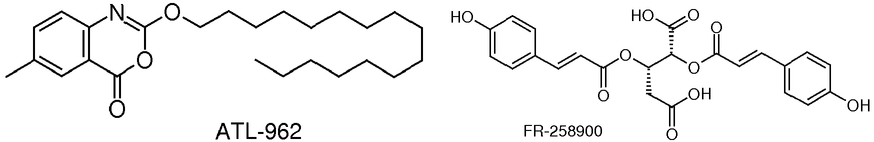

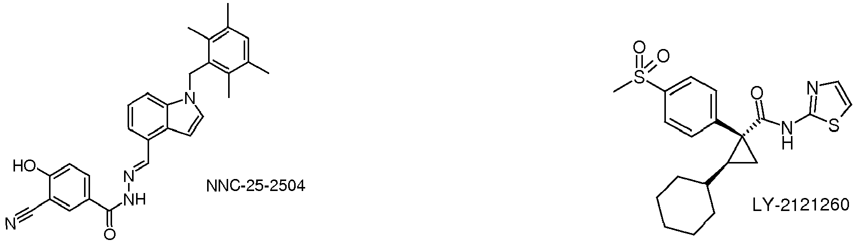

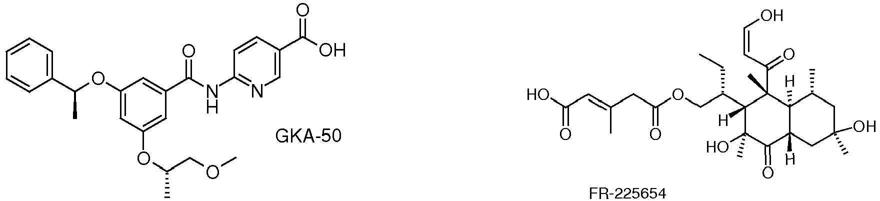

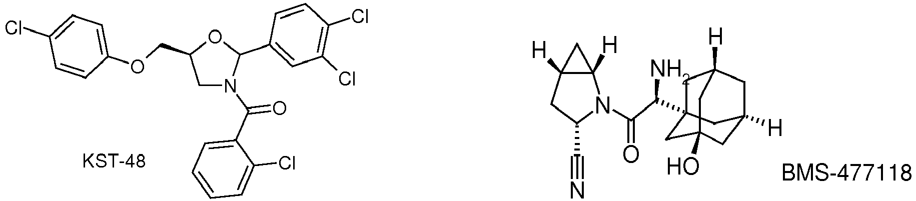

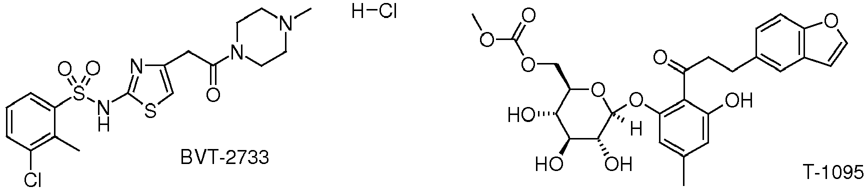

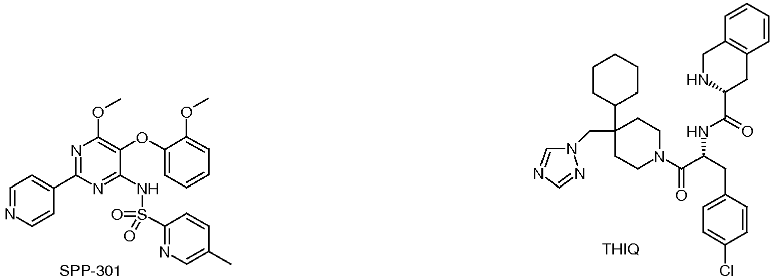

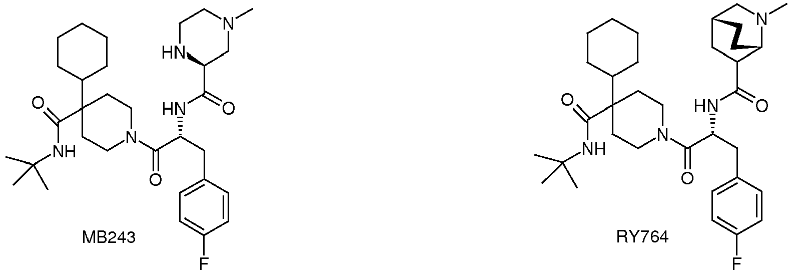

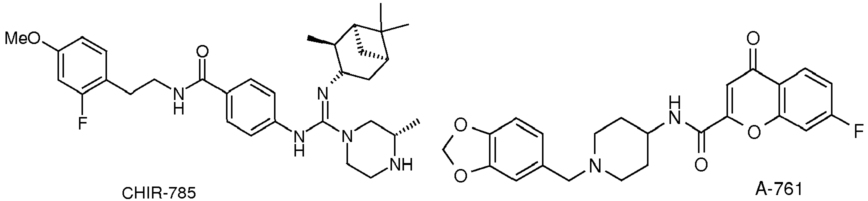

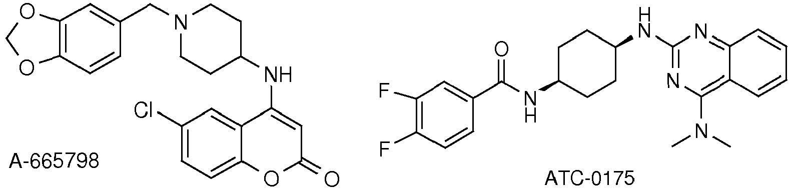

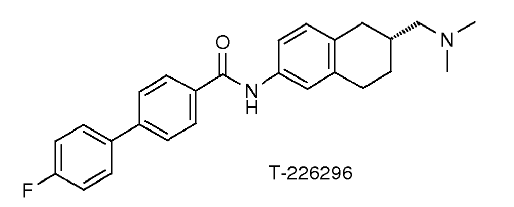

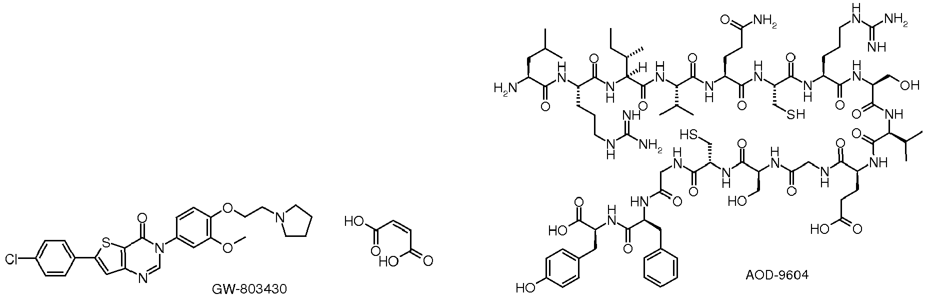

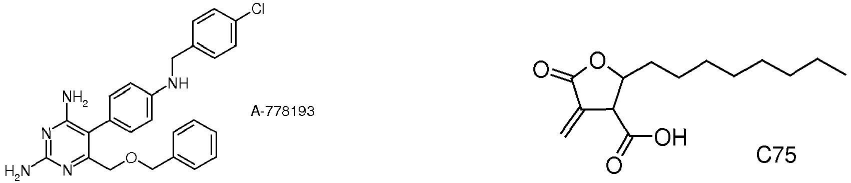

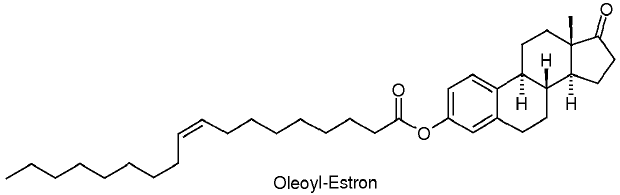

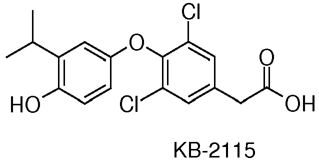

Die Wirksamkeit der erfindungsgemäßen Verbindungen der Formel I wurde an folgendem Enzymtestsystem geprüft:

### Test auf Hemmung der EL:

EL wird als sekretorisches Protein von rekombinanten Zell-Linien (CHO, HEK293) in hoher Konzentration in Zellkulturmedium abgegeben (konditioniertes Medium). Dieses wird nach Aufkonzentration als Enzymlösung eingesetzt.

### Assay auf EL-Aktivität

Zur Charakterisierung der enzymatischen Aktivität von endothelialer Lipase und der Wirkung von Inhibitoren wird das Phospholipase-spezifische Substrat 1,2-bis-(4,4-difluoro-5,7-dimethyl-4-bora-3a,4a-diaza-s-indacene-3-undecanoyl)-*sn*-glycero-3-phosphocholine, (Hersteller Molecular Probes) verwendet. Durch Hydrolyse der A1 Esterbindung dieses Phospholipids durch das Enzym wird eine mit dem Fluoreszenzfarbstoff Bodipy markierte Fettsäure freigesetzt, die nach Trennung durch Dünnschichtchromatographie auf einer HPTLC-Platte (Kieselgel 60, Merck) oder direkt im Reaktionsgefäß durch Messen der Fluoreszenz nachgewiesen werden kann. Zur Herstellung der Substratlösung werden 100 µg 1,2-bis-(4,4-difluoro-5,7-dimethyl-4-bora-3a,4a-diaza-s-indacene-3-undecanoyl)-*sn*-glycero-3-phospho-choline (Hersteller Molecular Probes) in 100µl DMSO gelöst und in 2,4 mg Tripalmitin (Sigma) in 393 µl Chloroform aufgenommen, welches 20mg/ ml DOP - Cholin (1,2-Dioleoyl-sn-glycero-3-phosphocholin) enthält. 39,3µl diesesLipidgemisches werden in ein frisches Reaktionsgefäß überführt und das Lösungsmittel abgedampft. Das Lipidgemisch wird in 4 ml 200 mM TRIS-HCl, 150 mM Natriumchlorid, pH = 7,4 durch zweimaliges Sonifizieren gelöst. Die anschließende Enzymreaktion erfolgt für 90 Minuten bei 37°C. Hierzu werden 20 µl der Substratlösung mit 2 µl Inhibitor entsprechender Konzentration (gelöst in 10% DMSO, zur Kontrolle wird 10%ige DMSO-Lösung verwendet) und 2 µl Enzymlösung (konditioniertes Medium) inkubiert. Im Anschluß werden 4 µl des Testansatzes auf eine HPTLC-Platte (Kieselgel 60, Merck) aufgetragen und der freigesetzte Fluoreszenzfarbstoff zum Nachweis mit einem Fließmittel (Diethylether : Petroleumbenzin : Essigsäure [78:22:1]) getrennt. Nach dem Abdampfen des Fließmittels wird die Platte in einen Fluoreszenz-Scanner eingelesen. Als Maß für die Enzymaktivität ist eine gesteigerte Freisetzung des Fluoreszenzfarbstoffes in der ungehemmten Reaktion zu beobachten.

In Abhängigkeit der verwendeten Inhibitorkonzentration ergibt sich eine Reduktion der enzymatischen Aktivität, die Inhibitiorkonzentration, bei welcher eine halbmaximale Enzymaktivität beobachtet wird, wird als IC₅₀ bezeichnet.

In diesen Tests zeigten die Verbindungen der Beispiele folgende IC₅₀ -Werte:

| Beispiel | IC₅₀ [µM] EL | Beispiel | IC₅₀ [µM] EL |
|---|---|---|---|
| 1 | 0.005 | 18 | 0,044 |
| 2 | 0.147 | 19 | 0,039 |
| 3 | 0,145 | 23 | 0,059 |
| 4 | 0.0005 | 24 | 0,143 |
| 5 | 0.008 | | |
| 6 | 0.143 | | |
| 7 | 0.083 | | |

### Andere Prüfmodelle

Anhand verschiedener Prüfmodelle kann die Eignung der erfindungsgemäßen Verbindungen als pharmazeutischer Wirkstoff getestet werden. Im Folgenden werden beispielhaft Beschreibungen solcher Prüfmodelle gegeben.

### Löslichkeiten in wässrigen Systemen

Ausreichende Löslichkeit einer Substanz in wässrigen Lösungsmittelsystemen ist eine wichtige Vorraussetzung für eine (reproduzierbare) pharmakologische Wirkung. Löslichkeiten in wässrigen Systemen können nach verschiedenen Verfahren bestimmt werden. Geeignet sind z. B. Fällungsverfahren aus Lösungen ("kinetische Löslichkeit") und Verfahren, die die Auflösung einer festen Probe bis zur Gleichgewichtseinstellung untersuchen ("thermodynamische Löslichkeit").

### a) Kinetische Löslichkeit

Auf einer 96-well Mikrotiterplatte wird eine DMSO-Lösung der Testverbindung (2,5 mM; 0,5 µL) zu 200 µL einer wässrigen Testlösung (z. B. Phosphate buffered Saline, 10x, 1 M, Sigma eingestellt auf 10 mM, pH 7,4) pipettiert und die Trübung bei der so erhaltenen theoretischen Konzentration der Testverbindung von 6,25 µM mittels eines Nephelometers (z. B. Nephelostar Galaxy, BMG Labtech) gemessen. Danach wird die Konzentration der Testverbindung in der wässrigen Testlösung durch Zugabe von weiterer DMSO-Lösung (2,5 mM; 0,5 µL) auf theoretisch 12,5 µM erhöht und die Trübungsmessung erneut durchgeführt. Weitere Zugaben von DMSO-Lösungen (1 µL, 2,5 mM; 0,5 µL, 10 mM; dann 9-mal 1 µL, 10 mM ergebend theoretische Konzentrationen von 25 µM, 50 µM, 100 µM, 150 µM, 200 µM, 250 µM, 300 µM, 350 µM, 400 µM, 450 µM und 500 µM) mit zwischenzeitlicher Trübungsmessung komplettieren den Messprozess.

Auswertung: Die Trübungswerte des Nephelometers werden gegen die theoretische Konzentration der Testverbindung in der wässrigen Testlösung aufgetragen. Sobald bei einer theoretischen Konzentration eine signifikante Trübung detektiert wird (z. B. 5-fach über dem Kontrollwert der wässrigen Testlösung), wird der darunter liegende Konzentrationswert als Löslichkeitsgrenze der Testverbindung in der Testlösung angegeben. Als maximal möglicher Messbereich ergeben sich damit die Werte <6,25 µM, 6,25 - 500 µM und >500 µM.

Bevorzugte erfindungsgemäße Verbindungen zeigen eine kinetische Löslichkeit im Phosphatpuffer (pH 7,4) von mindestens 12,5 µM; bevorzugter von mindestens 50 µM und noch stärker bevorzugt von mindestens 250 µM.

### b) Thermodynamische Löslichkeit

Mittels HPLC-UV-Messung einer Verdünnungsreihe der Testverbindung in DMSO (500 µM, 100 µM, 50 µM, 10 µM und 1 µM) wird die integrierte UV-Absorption in einer Kalibiergeraden linear mit der Konzentration korreliert. Die Testverbindung (500 µg) wird zusammen mit der wässrigen Testlösung (250 µL) in einem geschlossenen Gefäß (Fassungsvolumen: 1,5 mL) für 16 Stunden geschüttelt (Eppendorf Thermoschüttler, 1400 rpm, 25°C, Abdeckung als Lichtschutz). Anschließend wird die Probe bei maximaler Drehzahl zentrifugiert und der Überstand abschließend noch filtriert. Eine Probe des filtrierten Überstandes wird direkt mittels HPLC-UV-Messung (siehe oben) analysiert. Eine weitere Probe wird nach Verdünnen (1 Volumenteil Überstand, 39 Volumenteile Testlösung) analysiert.

Auswertung: Anhand der erstellten Kalibiergeraden wird aus den erhaltenen integrierten UV-Absorptionen der Überstandsproben die Konzentration der Testverbindung im unverdünnten Überstand berechnet und als Löslichkeit der Testverbindung in der jeweiligen wässrigen Testlösung angegeben.

Beispiele für wässrige Testlösungen sind entsalztes Wasser oder wässrige Phosphatpuffer mit verschiedenen pH-Werten (z. B. pH 1,2; pH 4,0; pH 6,8; pH 7,4; pH 9,0), die nach Standardverfahren aus der kommerziellen Lösung (Phosphate buffered saline, 10x, Sigma) hergestellt werden können durch Verdünnen bzw. Einstellen mit Phosphorsäure oder Natronlauge.

Bevorzugte erfindungsgemäße Verbindungen zeigen eine Löslichkeit im Phosphatpuffer (pH 7,4) von mindestens 12,5 µM; bevorzugter von mindestens 50 µM und noch stärker bevorzugt von mindestens 250 µM.

### Metabolische Stabilität

Die metabolische Stabilität wird bestimmt durch Inkubation der Testverbindung (5 µM) bei 37°C mit microsomalen Leberfraktionen (1 mg/mL Protein mit 0,1 % w/v BSA; 1 mM NADPH, 0,5% DMSO). Die Analyse bei 0 und 20 Minuten Inkubationszeit erfolgt mittels LCMS/MS. Weitere Beschreibungen des Testsystems und Referenzen zur experimentellen Durchführung finden sich bei Plant, N., Drug Discovery Today 2004, 9(7), 328-336 und Lau, Y.Y. et al., Pharmaceutical Res. 2002, 19(11), 1606-1610.

### Verfahren zur Herstellung

Die Herstellung der erfindungsgemäßen Verbindungen der allgemeinen Formel I erfolgt nach an und für sich bekannten Methoden z.B. durch Acylierung von substituierten, bzw. unsubstituierten Imidazolidin-derivaten mit Carbamoylchloriden III (Methode A), oder in zwei Stufen durch Umsetzung von Imidazolidin-derivate II mit Phosgen oder Äquivalenten wie Chlorcarbonsäuretrichlormethylester, Carbonsäureditrichlormethylester oder Chlorameisensäure-4-nitrophenylester und weiterer Umsetzung der erhaltenen Imidazolidin-carbonsäurederivate mit Aminen IV (Methode B). Ebenso können die Imidazolidin-derivate II auch mit den entsprechenden Isocyanaten V R1-N=C=O umgesetzt werden (Methode C). 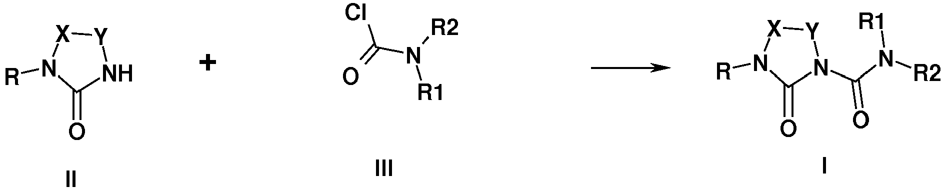 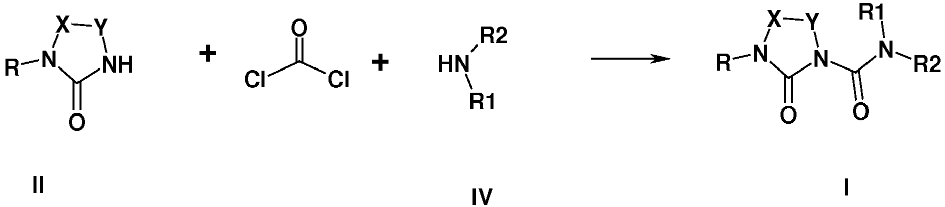 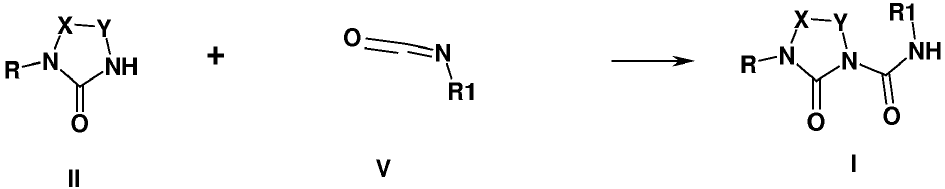 Die Reste R (ungleich Wasserstoff) können auch nachträglich durch Alkylierung der Verbindungen I (mit R =Wasserstoff) nach Literatur bekannten Verfahren eingeführt werden.
Reste R des Types -(C=O)-NR1aR2a können nach den oben genannten Methoden A, B oder C eingeführt werden. Dies kann durch Einsatz der Komponenten IVa (HNR1 aR2a) und Phosgen, bzw. III oder Va (O=C=NR1 a) mit mehr als 2-fachen Überschuß zu den Ausgangsverbindungen II geschehen.

Die nach oben beschriebenen Verfahren erhaltenen Verbindungen der Formel I können durch bekannte Trennverfahren, wie beispielsweise durch Kristallisation oder chromatographische Verfahren, getrennt werden. Beispielsweise können so monosubstituierte von disubstituierten Imidazolidin-derivaten getrennt werden.

Da bei diesen Reaktionen in der Regel Säuren freigesetzt werden, empfiehlt es sich zur Beschleunigung Basen wie Pyridin, Triethylamin, Natronlauge oder Alkalicarbonate zu zusetzen. Die Reaktionen können in weiten Temperaturbereichen durchgeführt werden. In der Regel hat es sich als vorteilhaft herausgestellt, bei 0°C bis zum Siedepunkt des verwendeten Lösungsmittels zu arbeiten. Als Lösemittel kommen beispielsweise Methylenchlorid, THF, DMF, Toluol, Essigester, n-Heptan, Dioxan, Diethylether oder Pyridin zum Einsatz. Wenn unter wasserfreien Bedingungen gearbeitet wird, haben sich auch starke Basen wie Lithiumhydrid, Natriumhydrid oder Kalium-tert.-butylat in aprotischen Lösungsmitteln wie THF oder DMF bewährt.
Die als Ausgangsverbindungen II eingesetzten Imidazolidin-derivate sind kommerziell erhältlich oder lassen sich nach Literatur bekannten Verfahren herstellen (A. Boeijen; J. W: A. Kruijtzer, R. M. J. Liskamp, Bioorg. Med. Chem. Lett. 1998 (8), 2375-2380; J. C. Hodges, S. Klutchko, US 5308853).

Die nachfolgend aufgeführten Beispiele dienen zur Erläuterung der Erfindung, ohne diese jedoch einzuschränken.
Die Identität der Verbindungen wurde durch Massenspektrometrie geprüft.

### Beispiele

### Beispiel 1:

### 3-Benzyl-2,5-dioxo-imidazolidin-1- carbonsäure hexylamid

1-Benzyl-imidazolidin-2,4-dion (100 mg, 0.526 mmol) und 1-Isocyanato-hexan (80.3 mg, 0.63 mmol) und bei Bedarf eine katalytische Menge 4-Dimethylaminopyridin wurden in 10 ml Toluol und 0.5 ml Pyridin gelöst und 6 h bei 115 °C gerührt. Anschließend wurden nochmals 80 mg 1-Isocyanato-hexan zugegeben und weitere 6 h bei 115 °C gerührt. Das Reaktionsgemisch wurde eingeengt und der Rückstand in Ethylacetat und H2O aufgenommen, die org. Phasen 2 mal mit Wasser gewaschen, eingeengt und über präparative HPLC (PR18, Acetonitril/Wasser 0.1% TFA) gereinigt. Ausbeute: 11.3 mg (7%), M+H+:318.14.

### Beispiel 2:

### 3-Benzyl-2,5-dioxo-imidazolidin-1-carbonsäure 2-methyl-benzylamid

Analog Beispiel 1 wurden 1-Benzyl-imidazolidin-2,4-dion (100 mg, 0.526 mmol) und 1-Isocyanatomethyl-2-methyl-benzol (92.9 mg, 0.63 mmol) umgesetzt. Ausbeute: 54 mg (30 %), M+H+:338.17.

### Beispiel 3:

### 3-Benzyl-2,5-dioxo-imidazolidin-1- carbonsäure (S)-indan-1-ylamid

Analog Beispiel 1 wurden 1-Benzyl-imidazolidin-2,4-dion (100 mg, 0.526 mmol) und (S)-1-Isocyanato-indan (100.4 mg, 0.63 mmol) umgesetzt. Ausbeute: 95 mg (52 %), M+H+: 350.19.

### Beispiel 4:

### 3-Benzyl-2,5-dioxo-imidazolidin-1- carbonsäure (R)-indan-1-ylamid

Analog Beispiel 1 wurden 1-Benzyl-imidazolidin-2,4-dion (100 mg, 0.526 mmol) und (R)-1-Isocyanato-indan (100.4 mg, 0.63 mmol) umgesetzt. Ausbeute: 48 mg (26 %), M+H+: 350.19.

### Beispiel 5:

### 3-Benzyl-2,5-dioxo-imidazolidin-1- carbonsäure (1,2,3,4-tetrahydro-naphthalin-1-yl)-amid

Analog Beispiel 1 wurden 1-Benzyl-imidazolidin-2,4-dion (100 mg, 0.526 mmol) und 1-Isocyanato-1,2,3,4-tetrahydro-naphthalin (109.3 mg, 0.63 mmol) umgesetzt. Ausbeute: 60 mg (31 %), M+H+: 364.16.

### Beispiel 6:

### 3-Benzyl-2,5-dioxo-imidazolidin-1- carbonsäure (2-thiophen-2-yl-et hyl)-amid

Analog Beispiel 1 wurden 1-Benzyl-imidazolidin-2,4-dion (100 mg, 0.526 mmol) und 2-(2-Isocyanato-ethyl)-thiophen (96.7 mg, 0.63 mmol) umgesetzt. Ausbeute: 53.5 mg (30 %), M+H+: 344.08.

### Beispiel 7:

### 3-Benzyl-2,5-dioxo-imidazolidin-1- carbonsäure heptylamid

Analog Beispiel 1 wurden 1-Benzyl-imidazolidin-2,4-dion (100 mg, 0.526 mmol) und 1-Isocyanato-heptan (89.1 mg, 0.63 mmol) umgesetzt. Ausbeute: 63.8 mg (37 %), M+H+: 332.20.

### Beispiel 8:

### 3-Benzyl-2,4-dioxo-imidazolidin-1- carbonsäure hexylamid

Analog Beispiel 1 wurden 3-Benzyl-imidazolidin-2,4-dion (100 mg, 0.526 mmol) und 1-Isocyanato-hexan (80.2 mg, 0.63 mmol) umgesetzt. Ausbeute: 19.3 mg (12 %), M+H+: 318.15.

### Beispiel 9:

### 3-Benzyl-2,4-dioxo-imidazolidin-1- carbonsäure 2-methyl-benzylamid

Analog Beispiel 1 wurden 3-Benzyl-imidazolidin-2,4-dion (100 mg, 0.526 mmol) und 1-Isocyanatomethyl-2-methyl-benzol (92.9 mg, 0.63 mmol) umgesetzt. Ausbeute: 75.3 mg (42 %), M+H+: 338.14.

### Beispiel 10:

### 3-Benzyl-2,4-dioxo-imidazolidin-1- carbonsäure heptylamid

Analog Beispiel 1 wurden 3-Benzyl-imidazolidin-2,4-dion (100 mg, 0.526 mmol) und 1-Isocyanato-heptan (89.1 mg, 0.63 mmol) umgesetzt. Ausbeute: 48 mg (28 %), M+H+: 332.17.

### Beispiel 11:

### 3-Benzyl-2,4-dioxo-imidazolidin-1- carbonsäure (2-thiophen-2-yl-ethyl)-amid

Analog Beispiel 1 wurden 3-Benzyl-imidazolidin-2,4-dion (100 mg, 0.526 mmol) und 2-(2-Isocyanato-ethyl)-thiophen (96.7 mg, 0.63 mmol) umgesetzt. Ausbeute: 34.5 mg (19 %), M+H+:

### Beispiel 12:

5,5-Dimethyl-2,4-dioxo-imidazolidin-1- carbonsäure hexylamid

Analog Beispiel 1 wurden 5,5-Dimethyl-3-morpholin-4-ylmethyl-imidazolidin-2,4-dion (100 mg, 0.44 mmol) und 1-Isocyanato-hexan (56 mg, 0.44 mmol) umgesetzt. Ausbeute: 8 mg (7 %), M+H+: 256.21.

### Beispiel 13:

### 3-(5,5-Dimethyl-2-oxo-tetrahydro-furan-3-yl)-5,5-dimethyl-2,4-dioxo-imidazolidin-1-carbonsäure hexylamid

Analog Beispiel 1 wurden 3-(5,5-Dimethyl-2-oxo-tetrahydro-furan-3-yl)-5,5-dimethyl-imidazolidin-2,4-dion (100 mg, 0.416 mmol) und 1-Isocyanato-hexan (52.9 mg, 0.416 mmol) umgesetzt. Ausbeute: 6 mg (4 %), M+H+: 368.25.

### Beispiel 14:

### 3-Benzyl-2,5-dioxo-imidazolidin-1- carbonsäure pentylamid

Analog Beispiel 1 wurden 1-Benzyl-imidazolidin-2,4-dion (100 mg, 0.526 mmol) und 1-Isocyanato-pentan (71.4 mg, 0.63 mmol) umgesetzt. Ausbeute: 15 mg (9 %), M+H+: 304.18.

### Beispiel 15:

### 3-Benzyl-2,5-dioxo-imidazolidin-1- carbonsäure cyclohexylamid

Analog Beispiel 1 wurden 1-Benzyl-imidazolidin-2,4-dion (100 mg, 0.526 mmol) und Isocyanato-cyclohexan (79 mg, 0.63 mmol) umgesetzt. Ausbeute: 22 mg (13 %), M+H+: 316.18.

### Beispiel 16:

### 3-Benzyl-2,5-dioxo-imidazolidin-1- carbonsäure cyclohexylmethyl-amid

Analog Beispiel 1 wurden 1-Benzyl-imidazolidin-2,4-dion (100 mg, 0.526 mmol) und Isocyanatomethyl-cyclohexan (87.8 mg, 0.63 mmol) umgesetzt. Ausbeute: 36.8 mg (21 %), M+H+: 330.18.

### Beispiel 17:

### 3-Benzyl-2,5-dioxo-imidazolidin-1- carbonsäure benzylamid

Analog Beispiel 1 wurden 1-Benzyl-imidazolidin-2,4-dion (100 mg, 0.526 mmol) und Isocyanatomethylbenzol (84 mg, 0.63 mmol) umgesetzt. Ausbeute: 39.8 mg (23 %), M+H+: 324.14.

### Beispiel 18:

### 1,3-Dioxo-hexahydro-imidazo[1,5-a]pyridin-2-carbonsäure hexylamid

### a) Tetrahydro-imidazo[1,5-a]pyridin-1,3-dion

Pipecolincarbonsäuremethylester Hydrochlorid (1 g, 5,567 mmol) wurde in Wasser gelöst und bei Raumtemperatur Kaliumcyanat (452 mg, 5,567 mmol) in 2 ml Wasser zugetropft. Nach 1 h Rühren bei Raumtemperatur wurde nochmals Kaliumcyanat (130 mg) in 2 ml Wasser zugetropft und 3 h weiter gerührt. Die Reaktionslösung wurde mit Salzsäure angesäuert, eingeengt und mit wenig Wasser verrührt. Der Feststoff wurde abgesaugt und getrocknet. Ausbeute: 191 mg (22 %), M+H+: 155.10. Bei wiederholter Aufarbeitung konnte weiteres Produkt isoliert werden.

### b) 1,3-Dioxo-hexahydro-imidazo[1,5-a]pyridin-2-carbonsäure hexylamid

Tetrahydro-imidazo[1,5-a]pyridin-1,3-dion (690 mg, 4,476 mmol) und Isocyanato-hexan (1,5 ml, 10,3 mmol, Zugabe in mehreren Portionen) wurden in 30 ml Dioxan 3 h bei 80 °C und 5 h bei 110 °C gerührt. Nach dem Einengen wurde über präparative HPLC (PR18, Acetonitril/Wasser 0.1% TFA) gereinigt. Ausbeute: 23 mg (2%), M+H+:282.20.

### Beispiel 19:

### 1,3-Dioxo-1,5,10,10a-tetrahydro-imidazo[1,5-b]isoquinolin-2-carbonsäure-hexylamid

Analog Beispiel 18 wurde 10,10a-Dihydro-5H-imidazo[1,5-b]isoquinolin-1,3-dion aus 1,2,3,4-Tetrahydro-isoquinolin-3- carbonsäure ethyl ester hergestellt (Ausbeute 85 %) und dann 1,48 mmol, wie in Beispiel 18 beschrieben, mit Isocyanato-hexan umgesetzt. Ausbeute: 61 mg (12%), M+H+:330.20.

### Beispiel 20:

### 1,3-Dioxo-hexahydro-imidazo[1,5-a]pyridin-2-carbonsäure-2-methyl-benzylamid

Analog Beispiel 18 wurde Tetrahydro-imidazo[1,5-a]pyridin-1,3-dion mit 1-Isocyanatomethyl-2-methyl-benzol umgesetzt. Ausbeute: 12%, M+H+: 302.23.

### Beispiel 21:

### 1,3-Dioxo-1,5,10,10a-tetrahydro-imidazo[1,5-b]isoquinolin-2-carbonsäure-(S)-indan-1-ylamid

Analog Beispiel 18 wurde 10,10a-Dihydro-5H-imidazo[1,5-b]isoquinolin-1,3-dion mit (S)-1-Isocyanato-indan umgesetzt. Ausbeute: 5 %, M+H+: 362.11.

### Beispiel 22:

### 1,3-Dioxo-1,5,10,10a-tetrahydro-imidazo[1,5-b]isoquinolin-2-carbonsäure-(R)-indan-1-ylamid

Analog Beispiel 18 wurde 10,10a-Dihydro-5H-imidazo[1,5-b]isoquinolin-1,3-dion mit (R)-1-Isocyanato-indan umgesetzt. Ausbeute: 6 %, M+H+: 362.13.

### Beispiel 23:

### 1,3-Dioxo-1,5,10,10a-tetrahydro-imidazo[1,5-b]isoquinolin-2-carbonsäure-(1,2,3,4-tetrahydro-naphthalin-1-yl)-amid

Analog Beispiel 18 wurde 10,10a-Dihydro-5H-imidazo[1,5-b]isoquinolin-1,3-dion mit 1-Isocyanato-1,2,3,4-tetrahydro-naphthalin umgesetzt. Ausbeute: 8 %, M+H+: 376.12.

### Beispiel 24:

### 1,3-Dioxo-1,5,10,10a-tetrahydro-imidazo[1,5-b]isoquinolin-2-carbonsäure-2-methyl-benzylamid

Analog Beispiel 18 wurde 10,10a-Dihydro-5H-imidazo[1,5-b]isoquinolin-1,3-dion mit 1-Isocyanatomethyl-2-methyl-benzol umgesetzt. Ausbeute: 2 %, M+H+: 350.12.

### Beispiel 25:

### 1,3-Dioxo-hexahydro-imidazo[1,5-a]pyridin-2-carbonsäure-(S)-1,2,3,4-tetrahydronaphthalin-1-yl)-amid

Analog Beispiel 18 wurde Tetrahydro-imidazo[1,5-a]pyridin-1,3-dion mit (S)-1-Isocyanato-1,2,3,4-tetrahydro-naphthalin umgesetzt. Ausbeute: 24%, M+H+: 328.16.

### Beispiel 26:

### 1,3-Dioxo-hexahydro-imidazo[1,5-a]pyridin-2-carbonsäure-(S)-indan-1-ylamid

Analog Beispiel 18 wurde Tetrahydro-imidazo[1,5-a]pyridin-1,3-dion mit (S)-1-Isocyanato-indan umgesetzt. Ausbeute: 9%, M+H+:.314.14.

### Beispiel 27:

### 1,3-Dioxo-hexahydro-imidazo[1,5-a]pyridin-2-carbonsäure-(R)-indan-1-ylamid

Analog Beispiel 18 wurde Tetrahydro-imidazo[1,5-a]pyridin-1,3-dion mit (R)-1-Isocyanato-indan umgesetzt. Ausbeute: 18%, M+H+: 314.14.

## Patentansprüche

1. Verbindung der allgemeinen Formel I 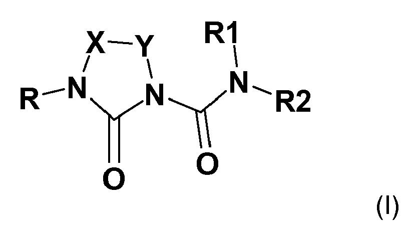 worin bedeuten:
X, Y gleich oder verschieden -C(R3)(R4)-, -(C=O)-, -(C=S), wobei mindestens ein X oder Y für -(C=O)- oder -(C=S)- steht; aber nicht beide gleichzeitig für -(C=O)- oder -(C=S)- stehen können; oder X und Y zusammen für C(R3)=C(R3) stehen;
R Wasserstoff, (C₁-C₅)-Haloalkyl, (CR5R6)ₘ-O(R7), (C₁-C₃)-Alkyloxy-(C₁-C₃)-Alkylen, Aryl, Heterocyclus, (C₁-C₄)-Alkylen-Aryl, (C₁-C₄)-Alkylen-Heteroaryl, (C₁-C₄)-Alkylen-(C₅-C₁₂)-Cycloalkyl, wobei Cycloalkyl, Aryl, Heterocyclus oder Heteroaryl durch F, Cl, Br, I, OH, CF₃, NO₂, CN, OCF₃, O-(C₁-C₆)-Alkyl, O-(C₁-C₄)-Alkoxy-(C₁-C₄)-Alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, (C₀-C₈)-Alkylen-Aryl, O-(C₀-C₈)-Alkylen-Aryl, S-Aryl, (C₀-C₈)-Alkylen-Heteroaryl, N(R5)(R6), SO₂-CH₃, SO₂-NH₂, SF₅, COOH, COO-(C₁-C₆)-Alkyl, CON(R5)(R6), N(R5)CO(R6), N(R5)SO₂(R6), CO(R5), (CR5R6)ₘ-O(R7), O-CO-N(R5)(R6), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R5)(R6) ein- oder mehrfach substituiert sein kann, wobei Aryl oder Heteroaryl wiederum durch
F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₂-C₆)-Alkinyl, N(R8)(R9), SO₂-CH₃, SF₅, COOH, COO-(C₁-C₆)-Alkyl, CON(R8)(R9), N(R8)CO(R9), N(R8)SO₂(R9), CO(R8), (CR8R9)_{m''}-O(R10), O-CO-N(R8)(R9), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R8)(R9) ein oder mehrfach substituiert sein kann;
m, m', m" 0, 1, 2, 3, 4, 5, 6;
R5, R6, R7, R8, R9, R10 gleich oder verschieden Wasserstoff, (C₁-C₈)-Alkyl;
oder -(C=O)-NR1aR2a; oder -(C=O)-O-R1b;
oder
R und X für X = -C(R3)(R4)- bilden ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem, deren einzelne Glieder der Ringsysteme durch ein bis drei Atome oder Atomgruppen aus der Reihe -CHR11-, -CR11R12-,-(C=R11)-, =C(R11)-, -NR11-, -C(=O)-, -O-, -S-, -SO-, -SO₂-, ersetzt sein können, mit der Maßgabe, dass zwei Einheiten aus der Reihe -O-, -S-, -SO-, -SO₂- nicht benachbart sein dürfen;
R11, R12 gleich oder verschieden Wasserstoff, (C₁-C₆)-Alkyl, Aryl, (C₃-C₁₂)-Cycloalkyl, (C₁-C₄)-Alkylen-Aryl, (C₁-C₃)-Alkylen-(C₃-C₁₂)-Cycloalkyl; wobei Aryl oder Cycloalkyl durch F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₂-C₆)-Alkinyl, N(R13)(R14), SO₂-CH₃, SO₂-NH₂, SF₅, COOH, COO-(C₁-C₆)-Alkyl, CON(R13)(R14), N(R13)CO(R14), N(R13)SO₂(R14), CO(R13), (CR13R14)ₙ-O(R15), O-CO-N(R13)(R14), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R13)(R14) substituiert sein kann;
n 0, 1, 2, 3, 4, 5, 6;
R13, R14, R15 gleich oder verschieden Wasserstoff, (C₁-C₈)-Alkyl;
R1, R1a, R1b gleich oder verschieden (C₅-C₁₆)-Alkyl, CH₂-Aryl, (C₁-C₂)-Alkylen-Heteroaryl, CH₂-(C₅-C₁₂)-Cycloalkyl, wobei Aryl, Heteroaryl oder Cycloalkyl durch F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, (C₀-C₈)-Alkylen-Aryl, O-(C₀-C₈)-Alkylen-Aryl, S-Aryl, (C₀-C₈)-Alkylen-Heteroaryl, N(R16)(R17), SO₂-CH₃, SO₂-NH₂, SF₅, COOH, COO-(C₁-C₆)-Alkyl, CON(R16)(R17), N(R16)CO(R17), N(R16)SO₂(R17), CO(R16), (CR16R17)ₒ-O(R18), O-CO-N(R16)(R17), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R16)(R17) ein- oder mehrfach substituiert sein kann, wobei Aryl oder Heteroaryl wiederum durch
F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₂-C₆)-Alkinyl, N(R19)(R20), SO₂-CH₃, SF₅, COOH, COO-(C₁-C₆)-Alkyl, CON(R19)(R20), N(R19)CO(R20), N(R19)SO₂(R20), CO(R19), (CR19R20)_{o'}-O(R21), O-CO-N(R19)(R20), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R19)(R20) ein oder mehrfach substituiert sein kann;
o, o' 0, 1, 2, 3, 4, 5, 6;
R16, R17, R18, R19, R20, R21 gleich oder verschieden Wasserstoff, (C₁-C₈)-Alkyl;
oder ein Rest der Formel la 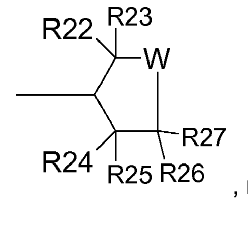 mit
W -C(R28)(R29)-, -C(R28)(R29)-C(R28a)(R29a)-, -C(R28)(R29)-O-;
R22, R23, R24, R25, R26, R27, R28, R29, R28a, R29a gleich oder verschieden Wasserstoff, F, Cl, Br, I, OH, CF₃, NO₂, CN, OCF₃, SF₅, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₃-C₈)-Cycloalkenyl, (C₂-C₆)-Alkinyl, N(R30)(R31), SO₂-CH₃, COOH, COO-(C₁-C₆)-Alkyl, CON(R30)(R31), N(R30)CO(R31), N(R30)SO₂(R31), CO(R30), (CR30R31)ₚ-O(R32), O-CO-N(R30)(R31), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R30)(R31);
p 0, 1, 2, 3, 4, 5, 6;
R30, R31, R32 gleich oder verschieden Wasserstoff, (C₁-C₆)-Alkyl;
oder
R22 und R28 oder R23 und R29 zusammen mit dem sie tragenden Kohlenstoffatomen bilden ein monocyclisches, 5 oder 6 gliederiges gesättigtes, teilweise ungesättigtes oder aromatisches Ringsystem, dessen einzelne Glieder durch -CHR33-, -CR33R34-, =(C-R33)- substituiert sein können;
oder
R24 und R26, oder R25 und R27 zusammen mit dem sie tragenden Kohlenstoffatomen bilden ein monocyclisches, 5 oder 6 gliederiges gesättigtes, teilweise ungesättigtes oder ein aromatisches Ringsystem, dessen einzelne Glieder durch -CHR33-, -CR33R34-, =(C-R33)- substituiert sein können;
R33, R34 gleich oder verschieden, F, Cl, Br, I, OH, CF₃, NO₂, CN, OCF₃, SF₅, O-(C₁-C₆)-Alkyl, O-(C₁-C₄)-Alkoxy-(C₁-C₄)-Alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, N(R35)(R36), SO₂-CH₃, COOH, COO-(C₁-C₆)-Alkyl, CON(R35)(R36), N(R35)CO(R36), N(R35)SO₂(R36), CO(R35), (CR35R36)_{q}-O(R37), O-CO-N(R35)(R36), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R35)(R36);
q 0, 1, 2, 3, 4, 5, 6;
R35, R36, R37 gleich oder verschieden Wasserstoff, (C₁-C₆)-Alkyl,
R2, R2a gleich oder verschieden Wasserstoff; (C₁-C₈)-Alkyl;
R3, R4 gleich oder verschieden Wasserstoff, (C₁-C₆)-Alkyl, Benzyl;
die tautomeren Formen der Verbindung sowie deren physiologisch verträgliche Salze;
mit der Maßgabe, dass wenn X= CH₂, Y=CO, R= Methyl, R2=H, R1 nicht Pentyl, CH₂-phenyl, -CH₂-(2-Cl-phenyl), Cyclohexyl, -(2-Methyl-Cyclohexyl) bedeutet.

2. Verbindung der Formel I nach Anspruch 1, worin R2 Wasserstoff ist.

3. Verbindung der Formel I nach Ansprüchen 1 oder 2, worin
Y -(C=O)- und
X -C(R3)(R4)-;
oder
X -(C=O)- und
Y -C(R3(R4)-;
R Wasserstoff, (C₁-C₃)-Haloalkyl, (CR5R6)ₘ-O(R7), Phenyl, Heterocyclus, (C₁-C₄)-Alkylen-Phenyl, (C₁-C₄)-Alkylen-Heteroaryl, (C₁-C₄)-Alkylen-(C₅-C₁₂)-Cycloalkyl, wobei Cycloalkyl, Phenyl, Heterocyclus oder Heteroaryl durch F, Cl, Br, I, OH, CF₃, NO₂, CN, OCF₃, O-(C₁-C₆)-Alkyl, O-(C₁-C₄)-Alkoxy-(C₁-C₄)-Alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, (C₀-C₈)-Alkylen-Aryl, O-(C₀-C₈)-Alkylen-Aryl, S-Aryl, (C₀-C₈)-Alkylen-Heteroaryl, N(R5)(R6), SO₂-CH₃, SO₂-NH₂, SF₅, COOH, COO-(C₁-C₆)-Alkyl, CON(R5)(R6), N(R5)CO(R6), N(R5)SO₂(R6), CO(R5), (CR5R6)_{m'}-O(R7), O-CO-N(R5)(R6), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R5)(R6) ein- oder mehrfach substituiert sein kann, wobei Aryl oder Heteroaryl wiederum durch
F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₂-C₆)-Alkinyl, N(R8)(R9), SO₂-CH₃, SF₅, COOH, COO-(C₁-C₆)-Alkyl, CON(R8)(R9), N(R8)CO(R9), N(R8)SO₂(R9), CO(R8), (CR8R9)_{m''}-O(R10), O-CO-N(R8)(R9), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R8)(R9) ein oder mehrfach substituiert sein kann;
m, m', m'' 0, 1, 2, 3, 4, 5, 6;
R5, R6, R7, R8, R9, R10 gleich oder verschieden Wasserstoff, (C₁-C₈)-Alkyl;
oder -(C=O)-NR1aR2a; oder -(C=O)-O-R1b;
oder
R und X für X = -C(R3)(R4)- bilden ein monocyclisches, gesättigtes 5- bis 7-gliedriges Ringsystem oder ein bicyclisches teilweise ungesättigtes 8-bis 14 gliedriges Ringsystem, dessen einzelne Glieder durch ein bis drei Atome oder Atomgruppen aus der Reihe -CHR11-, -CR11R12-,-(C=R11)-, -NR11-, -C(=O)-, -O-, ersetzt sein können, mit der Maßgabe, dass zwei Einheiten aus der Reihe -O- nicht benachbart sein dürfen;
R11, R12 gleich oder verschieden Wasserstoff, (C₁-C₆)-Alkyl, Phenyl, (C₃-C₁₂)-Cycloalkyl, (C₁-C₄)-Alkylen-Phenyl, (C₁-C₃)-Alkylen-(C₃-C₁₂)-Cycloalkyl; wobei Phenyl oder Cycloalkyl durch F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₂-C₆)-Alkinyl, N(R13)(R14), SO₂-CH₃, SO₂-NH₂, SF₅, COOH, COO-(C₁-C₆)-Alkyl, CON(R13)(R14), N(R13)CO(R14), N(R13)SO₂(R14), CO(R13), (CR13R14)ₙ-O(R15), O-CO-N(R13)(R14), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R13)(R14) substituiert sein kann;
n 0, 1, 2, 3, 4, 5, 6;
R13, R14, R15 unabhängig voneinander Wasserstoff, (C₁-C₈)-Alkyl;
R1, R1a, R1b gleich oder verschieden (C₅-C₁₂)-Alkyl, CH₂-Phenyl, (C₁-C₂)-Alkylen-Heteroaryl, -CH₂-(C₅-C₁₂)-Cycloalkyl, (C₅-C₆)-Cycloalkyl, wobei Phenyl, Heteroaryl oder Cycloalkyl durch F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, (C₀-C₈)-Alkylen-Phenyl, O-(C₀-C₈)-Alkylen-Phenyl, S-Phenyl, (C₀-C₈)-Alkylen-Heteroaryl, N(R16)(R17), SO₂-CH₃, SO₂-NH₂, SF₅, COOH, COO-(C₁-C₆)-Alkyl, CON(R16)(R17), N(R16)CO(R17), N(R16)SO₂(R17), CO(R16), (CR16R17)ₒ-O(R18), O-CO-N(R16)(R17), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R16)(R17) ein- oder mehrfach substituiert sein kann, wobei Phenyl oder Heteroaryl wiederum durch
F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₂-C₆)-Alkinyl, N(R19)(R20), SO₂-CH₃, SF₅, COOH, COO-(C₁-C₆)-Alkyl, CON(R19)(R20), N(R19)CO(R20), N(R19)SO₂(R20), CO(R19), (CR19R20)_{o'}-O(R21), O-CO-N(R19)(R20), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R19)(R20) ein oder mehrfach substituiert sein kann;
o, o' 0, 1, 2, 3, 4, 5, 6;
R16, R17, R18, R19, R20, R21 gleich oder verschieden Wasserstoff, (C₁-C₈)-Alkyl;
oder ein Rest der Formel Ib 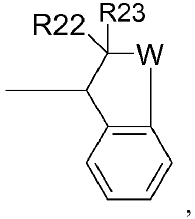 bedeuten.

4. Verbindung der Formel I gemäß Ansprüchen 1 bis 3, worin
Y -(C=O)- und
X -C(R3)(R4)-;
oder
X -(C=O)- und
Y -C(R3(R4)-;
R Wasserstoff, (CR5R6)ₘ-O(R7), Phenyl, CH₂-Phenyl, wobei Phenyl durch F, Cl, Br, CF₃, O-(C₁-C₆)-Alkyl, (C₁-C₆)-Alkyl, O-(C₂-C₄)-Haloalkyl, N(R5)(R6), SO₂-CH₃, SO₂-NH₂, COOH, COO-(C₁-C₆)-Alkyl, CON(R5)(R6), CO(R5), (CR5R6)_{m'}-O(R7) ein- oder zweifach substituiert sein kann,
m, m' 0, 1, 2, 3;
R5, R6, R7 gleich oder verschieden Wasserstoff, (C₁-C₈)-Alkyl;
oder
R und X für X = -C(R3)(R4)- bilden ein monocyclisches, gesättigtes 6-gliedriges Ringsystem oder ein bicyclisches teilweise ungesättigtes 9- bis 11 gliedriges Ringsystem, dessen einzelne Glieder durch ein bis zwei Atome oder Atomgruppen aus der Reihe -CHR11-, -CR11R12-,-(C=R11)-, =C(R11)- ersetzt sein können;
R11, R12 gleich oder verschieden Wasserstoff, (C₁-C₆)-Alkyl;
R1 (C₅-C₈)-Alkyl, -CH₂-Phenyl, (C₁-C₂)-Alkylen-Heteroaryl, wobei Heteroaryl ausgewählt ist aus der Gruppe Thiophen, Benzothiophen, Pyridin, Pyrazol, -CH₂-(C₅-C₇)-Cycloalkyl, (C₅-C₆)-Cycloalkyl, wobei Phenyl, Heteroaryl oder Cycloalkyl durch F, Cl, Br, OH, CF₃, NO₂, CN, OCF₃, O-(C₁-C₆)-Alkyl, (C₁-C₆)-Alkyl, (C₀-C₈)-Alkylen-Phenyl, O-(C₀-C₈)-Alkylen-Phenyl, (C₀-C₈)-Alkylen-Heteroaryl, N(R16)(R17), SO₂-CH₃, SO₂-NH₂, COOH, COO-(C₁-C₆)-Alkyl, CON(R16)(R17), CO(R16), (CR16R17)ₒ-O(R18) ein- oder mehrfach substituiert sein kann, wobei Phenyl oder Heteroaryl wiederum durch
F, Cl, Br, OH, CF₃, NO₂, CN, OCF₃, O-(C₁-C₆)-Alkyl, (C₁-C₆)-Alkyl, COOH, COO-(C₁-C₆)-Alkyl, CON(R19)(R20), CO(R19), (CR19R20)_{o'}-O(R21) ein oder mehrfach substituiert sein kann;
o, o' 0, 1, 2, 3;
R16, R17, R18, R19, R20, R21 gleich oder verschieden Wasserstoff, (C₁-C₈)-Alkyl;
oder ein Rest aus der Gruppe 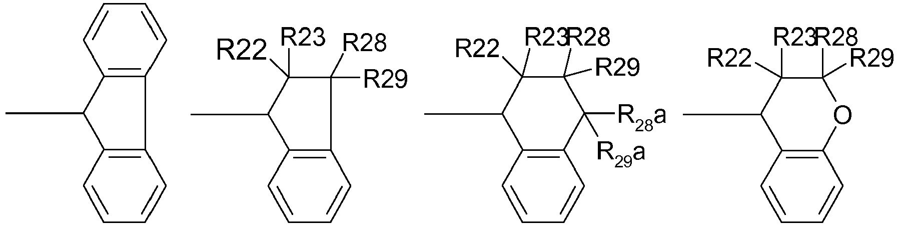 R22, R23, R28, R29, R28a, R29a gleich oder verschieden Wasserstoff, (C₁-C₆)-Alkyl, bevorzugt Wasserstoff und Methyl;
R2 Wasserstoff;
R3, R4 gleich oder verschieden Wasserstoff, Methyl;
bedeuten.

5. Verbindung der Formel I gemäß Ansprüchen 1 bis 4, worin
Y -(C=O)- und
X -C(R3)(R4)-;
oder
X -(C=O)- und
Y -C(R3(R4)-;
R Wasserstoff, (CR5R6)ₘ-O(R7), -CH₂-Phenyl, wobei Phenyl durch F, Cl, Br, CF₃, O-(C₁-C₆)-Alkyl, (C₁-C₆)-Alkyl, O-(C₂-C₄)-Haloalkyl, COOH, COO-(C₁-C₆)-Alkyl, CON(R5)(R6), CO(R5), (CR5R6)_{m'}-O(R7) ein- oder zweifach substituiert sein kann,
m, m' 0, 1, 2, 3;
R5, R6, R7 gleich oder verschieden Wasserstoff, (C₁-C₄)-Alkyl;
oder
R und X für X = -C(R3)(R4)- bilden ein monocyclisches, gesättigtes 6-gliedriges Ringsystem, an welches ein Benzolkern kondensiert sein kann, deren einzelne Glieder der Ringsysteme durch ein bis zwei Atomgruppen aus der Reihe -CHR11-, -CR11 R12-, -(C=R11)-, =C(R11)- ersetzt sein können;
R11, R12 gleich oder verschieden Wasserstoff, (C₁-C₆)-Alkyl;
R1 (C₅-C₈)-Alkyl, -CH₂-Phenyl, (C₁-C₂)-Alkylen-Heteroaryl, wobei Heteroaryl ausgewählt ist aus der Gruppe Thiophen, Benzothiophen,-CH₂-Cyclohexyl, Cyclohexyl, wobei Phenyl, Heteroaryl oder Cyclohexyl durch F, Cl, OH, CF₃, OCF₃, O-(C₁-C₆)-Alkyl, (C₁-C₆)-Alkyl, (C₀-C₁)-Alkylen-Phenyl, O-Phenyl, (C₀-C₁)-Alkylen-Heteroaryl, N(R16)(R17), COOH, COO-(C₁-C₆)-Alkyl, CON(R16)(R17), CO(R16), (CR16R17)ₒ-O(R18) ein- oder zweifach substituiert sein kann, wobei Phenyl oder Heteroaryl wiederum durch
F, Cl, OH, CF₃, O-(C₁-C₆)-Alkyl, (C₁-C₆)-Alkyl, COOH, COO-(C₁-C₆)-Alkyl, CON(R19)(R20), CO(R19), (CR19R20)_{o'}-O(R21) ein oder zweifach substituiert sein kann;
o,o' 0, 1, 2, 3;
R16, R17, R18, R19, R20, R21 gleich oder verschieden Wasserstoff, (C₁-C₈)-Alkyl;
oder ein Rest aus der Gruppe 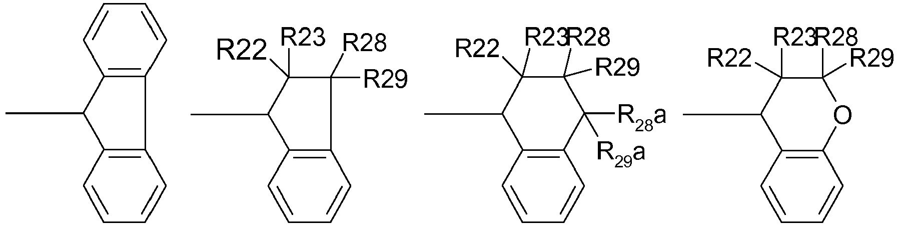 R22, R23, R28, R29, R28a, R29a gleich oder verschieden Wasserstoff, (C₁-C₆)-Alkyl, bevorzugt Wasserstoff und Methyl;
R2 Wasserstoff;
R3, R4 gleich oder verschieden Wasserstoff, Methyl;
bedeuten.

6. Verbindung der Formel I gemäß Ansprüchen 1 bis 5, worin
Y -(C=O)- und
X -C(R3)(R4)-;
oder
X -(C=O)- und
Y -C(R3)(R4)-;
R Wasserstoff, HO-CH₂-, Benzyl,
oder
R und X für X = -C(-R3)(R4)- bilden -CH₂-CH₂-CH₂-CH₂- oder 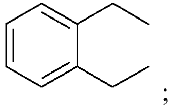
R1 Pentyl, Hexyl, Heptyl, Cyclohexyl, -CH₂-Cyclohexyl, -CH₂-Phenyl, -CH₂-Thiophen, -CH₂-CH₂-Thiophen, wobei Cyclohexyl, Phenyl oder Thiophen durch Methyl substituiert sein kann; 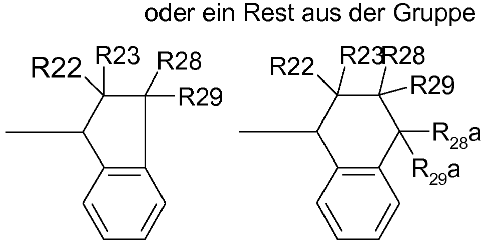
R22, R23, R28, R29, R28a, R29a Wasserstoff;
R2 Wasserstoff;
R3, R4 gleich oder verschieden Wasserstoff, Methyl;
bedeuten.

7. Arzneimittel enthaltend eine oder mehrere Verbindungen der Formel I gemäß den Ansprüchen 1 bis 6.

8. Arzneimittel enthaltend eine oder mehrere Verbindungen der Formel I 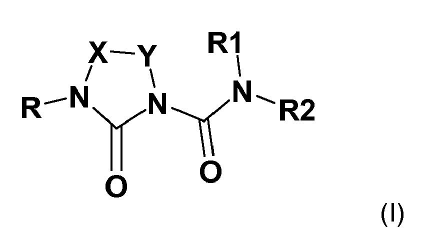 worin bedeuten:
X, Y gleich oder verschieden -C(R3)(R4)-, -(C=O)-, -(C=S), wobei mindestens ein X oder Y für -(C=O)- oder -(C=S)- steht; aber nicht beide gleichzeitig für -(C=O)- oder -(C=S)- stehen können; oder X und Y zusammen für C(R3)=C(R3) stehen;
R Wasserstoff, (C₁-C₈)-Alkyl, (C₁-C₅)-Haloalkyl, (CR5R6)ₘ-O(R7), (C₁-C₃)-Alkyloxy-(C₁-C₃)-Alkylen, Aryl, Heterocyclus, (C₁-C₄)-Alkylen-Aryl, (C₁-C₄)-Alkylen-Heteroaryl, (C₁-C₄)-Alkylen-(C₅-C₁₂)-Cycloalkyl, wobei Cycloalkyl, Aryl, Heterocyclus oder Heteroaryl durch F, Cl, Br, I, OH, CF₃, NO₂, CN, OCF₃, O-(C₁-C₆)-Alkyl, O-(C₁-C₄)-Alkoxy-(C₁-C₄)-Alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, (C₀-C₈)-Alkylen-Aryl, O-(C₀-C₈)-Alkylen-Aryl, S-Aryl, (C₀-C₈)-Alkylen-Heteroaryl, N(R5)(R6), SO₂-CH₃, SO₂-NH₂, SF₅, COOH, COO-(C₁-C₆)-Alkyl, CON(R5)(R6), N(R5)CO(R6), N(R5)SO₂(R6), CO(R5), (CR5R6)_{m'}-O(R7), O-CO-N(R5)(R6), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R5)(R6) ein- oder mehrfach substituiert sein kann, wobei Aryl oder Heteroaryl wiederum durch
F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₂-C₆)-Alkinyl, N(R8)(R9), SO₂-CH₃, SF₅, COOH, COO-(C₁-C₆)-Alkyl, CON(R8)(R9), N(R8)CO(R9), N(R8)SO₂(R9), CO(R8), (CR8R9)_{m''}-O(R10), O-CO-N(R8)(R9), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R8)(R9) ein oder mehrfach substituiert sein kann;
m, m', m" 0, 1, 2, 3, 4, 5, 6;
R5, R6, R7, R8, R9, R10 gleich oder verschieden Wasserstoff, (C₁-C₈)-Alkyl;
oder -(C=O)-NR1aR2a;
oder -(C=O)-O-R1b;
oder
R und X für X = -C(R3)(R4)- bilden ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem, deren einzelne Glieder der Ringsysteme durch ein bis drei Atome oder Atomgruppen aus der Reihe -CHR11-, -CR11R12-,-(C=R11)-, =C(R11)-, -NR11-, -C(=O)-, -O-, -S-, -SO-, -SO₂-, ersetzt sein können, mit der Maßgabe, dass zwei Einheiten aus der Reihe -O-, -S-, -SO-, -SO₂- nicht benachbart sein dürfen;
R11, R12 gleich oder verschieden Wasserstoff, (C₁-C₆)-Alkyl, Aryl, (C₃-C₁₂)-Cycloalkyl, (C₁-C₄)-Alkylen-Aryl, (C₁-C₃)-Alkylen-(C₃-C₁₂)-Cycloalkyl; wobei Aryl oder Cycloalkyl durch F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₂-C₆)-Alkinyl, N(R13)(R14), SO₂-CH₃, SO₂-NH₂, SF₅, COOH, COO-(C₁-C₆)-Alkyl, CON(R13)(R14), N(R13)CO(R14), N(R13)SO₂(R14), CO(R13), (CR13R14)ₙ-O(R15), O-CO-N(R13)(R14), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R13)(R14) substituiert sein kann;
n 0, 1, 2, 3, 4, 5, 6;
R13, R14, R15 gleich oder verschieden Wasserstoff, (C₁-C₈)-Alkyl;
R1, R1a, R1b gleich oder verschieden (C₅-C₁₆)-Alkyl, CH₂-Aryl, (C₁-C₂)-Alkylen-Heteroaryl, CH₂-(C₅-C₁₂)-Cycloalkyl, wobei Aryl, Heteroaryl oder Cycloalkyl durch F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, (C₀-C₈)-Alkylen-Aryl, O-(C₀-C₈)-Alkylen-Aryl, S-Aryl, (C₀-C₈)-Alkylen-Heteroaryl, N(R16)(R17), SO₂-CH₃, SO₂-NH₂, SF₅, COOH, COO-(C₁-C₆)-Alkyl, CON(R16)(R17), N(R16)CO(R17), N(R16)SO₂(R17), CO(R16), (CR16R17)ₒ-O(R18), O-CO-N(R16)(R17), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R16)(R17) ein- oder mehrfach substituiert sein kann, wobei Aryl oder Heteroaryl wiederum durch
F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₂-C₆)-Alkinyl, N(R19)(R20), SO₂-CH₃, SF₅, COOH, COO-(C₁-C₆)-Alkyl, CON(R19)(R20), N(R19)CO(R20), N(R19)SO₂(R20), CO(R19), (CR19R20)_{o'}-O(R21), O-CO-N(R19)(R20), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R19)(R20) ein oder mehrfach substituiert sein kann;
o, o' 0, 1, 2, 3, 4, 5, 6;
R16, R17, R18, R19, R20, R21 gleich oder verschieden Wasserstoff, (C₁-C₈)-Alkyl;
oder ein Rest der Formel la 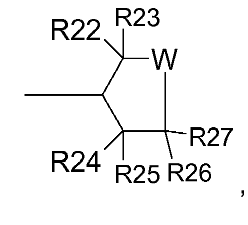 mit
W -C(R28)(R29)-, -C(R28)(R29)-C(R28a)(R29a)-, -C(R28)(R29)-O-;
R22, R23, R24, R25, R26, R27, R28, R29, R28a, R29a gleich oder verschieden Wasserstoff, F, Cl, Br, I, OH, CF₃, NO₂, CN, OCF₃, SF₅, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₃-C₈)-Cycloalkenyl, (C₂-C₆)-Alkinyl, N(R30)(R31), SO₂-CH₃, COOH, COO-(C₁-C₆)-Alkyl, CON(R30)(R31), N(R30)CO(R31), N(R30)SO₂(R31), CO(R30), (CR30R31)ₚ-O(R32), O-CO-N(R30)(R31), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R30)(R31);
p 0, 1, 2, 3, 4, 5, 6;
R30, R31, R32 gleich oder verschieden Wasserstoff, (C₁-C₆)-Alkyl;
oder
R22 und R28 oder R23 und R29 zusammen mit dem sie tragenden Kohlenstoffatomen bilden ein monocyclisches, 5 oder 6 gliederiges gesättigtes, teilweise ungesättigtes oder aromatisches Ringsystem, dessen einzelne Glieder durch -CHR33-, -CR33R34-, =(C-R33)- substituiert sein können;
oder
R24 und R26, oder R25 und R27 zusammen mit dem sie tragenden Kohlenstoffatomen bilden ein monocyclisches, 5 oder 6 gliederiges gesättigtes, teilweise ungesättigtes oder ein aromatisches Ringsystem, dessen einzelne Glieder durch -CHR33-, -CR33R34-, =(C-R33)- substituiert sein können;
R33, R34 gleich oder verschieden, F, Cl, Br, I, OH, CF₃, NO₂, CN, OCF₃, SF₅, O-(C₁-C₆)-Alkyl, O-(C₁-C₄)-Alkoxy-(C₁-C₄)-Alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, N(R35)(R36), SO₂-CH₃, COOH, COO-(C₁-C₆)-Alkyl, CON(R35)(R36), N(R35)CO(R36), N(R35)SO₂(R36), CO(R35), (CR35R36)_{q}-O(R37), O-CO-N(R35)(R36), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R35)(R36);
q 0, 1, 2, 3, 4, 5, 6;
R35, R36, R37 gleich oder verschieden Wasserstoff, (C₁-C₆)-Alkyl;
R2, R2a gleich oder verschieden Wasserstoff; (C₁-C₈)-Alkyl;
R3, R4 gleich oder verschieden Wasserstoff, (C₁-C₆)-Alkyl, Benzyl;
die tautomeren Formen der Verbindung sowie deren physiologisch verträgliche Salze;
und als weiteren Wirkstoff eine oder mehrere
Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATPabhängigen Kaliumkanal der Betazellen wirkende Wirkstoffe, CART-Agonisten, NPY-Agonisten, MC4-Agonisten, Orexin-Antgonisten, H3-Agonisten, TNF-Agonisten, CRF-Antagonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Serotonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

9. Verwendung der Verbindungen der Formel I gemäß Anspruch 8 zur Herstellung eines Medikaments zur Behandlung und/oder Prävention von Störungen des Fettsäurestoffwechsels und Glucoseverwertungsstörungen.

10. Verwendung der Verbindungen der Formel I gemäß Anspruch 8 zur Herstellung eines Medikaments zur Behandlung und/oder Prävention von Dyslipidämien und deren Folgen.

11. Verwendung der Verbindungen der Formel I gemäß Anspruch 8 zur Herstellung eines Medikaments zur Behandlung und/oder Prävention von Zuständen, die mit dem Metabolischen Syndrom assoziert sind, sowie bei Störungen, bei denen Insulin Resistenz eine Rolle spielt, oder zur Behandlung und/oder Prävention von Diabetes mellitus und der damit verbundenen Folgeerkrankungen.

12. Verwendung der Verbindungen der Formel I gemäß Anspruch 8 zur Herstellung eines Medikaments zur Behandlung und/oder Prävention von Zuständen, die mit erniedrigtem HDL-Spiegel verbunden sind.

13. Verwendung der Verbindungen der Formel I gemäß Anspruch 8 zur Herstellung eines Medikaments zur Behandlung und/oder Prävention von atherosklerotischen Erkrankungen.

14. Verwendung der Verbindungen der Formel I gemäß Anspruch 8 in Kombination mit mindestens einem weiteren Wirkstoff zur Herstellung eines Medikaments zur Behandlung und/oder Prävention von Störungen, bei denen Insulin Resistenz eine Rolle spielt.

15. Verfahren zur Herstellung eines Arzneimittels enthaltend eine oder mehrere der Verbindungen der Formel I gemäß Anspruch 8 **dadurch gekennzeichnet, dass** diese mit einem pharmazeutisch geeigneten Träger vermischt wird und diese Mischung in eine für die Verabreichung geeignete Form gebracht wird.

## Claims

1. A compound of the formula I 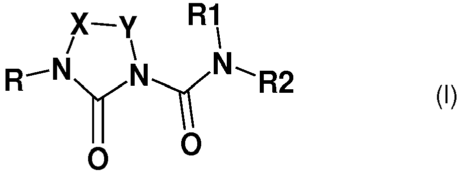 in which the meanings are:
X, Y identically or differently -C(R3)(R4)-, -(C=O)-, -(C=S), where at least one X or Y is -(C=O)- or -(C=S)-; but cannot both simultaneously be -(C=O)- or -(C=S)-; or X and Y together are C(R3)=C(R3);
R hydrogen, (C₁-C₅)-haloalkyl, (CR5R6)ₘ-O(R7), (C₁-C₃)-alkyloxy-(C₁-C₃)-alkylene, aryl, heterocycle, (C₁-C₄)-alkylene-aryl, (C₁-C₄)-alkylene-heteroaryl, (C₁-C₄)-alkylene-(C₅-C₁₂)-cycloalkyl, where cycloalkyl, aryl, heterocycle or heteroaryl may be substituted one or more times by F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₃-C₈)-cycloalkyl, O-(C₃-C₈)-cycloalkyl, (C₂-C₆)-alkynyl, (C₀-C₈)-alkylene-aryl, O-(C₀-C₈)-alkylene-aryl, S-aryl, (C₀-C₈)-alkylene-heteroaryl, N(R5)(R6), SO₂-CH₃, SO₂-NH₂, SF₅, COOH, COO-(C₁-C₆)-alkyl, CON(R5)(R6), N(R5)CO(R6), N(R5)SO₂(R6), CO(R5), (CR5R6)ₘ-O(R7), O-CO-N(R5)(R6), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R5)(R6), where aryl or heteroaryl may in turn be substituted one or more times by
F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, (C₃-C₈)-cycloalkyl, O-(C₃-C₈)-cycloalkyl, (C₂-C₆)-alkynyl, N(R8)(R9), SO₂-CH₃, SF₅, COOH, COO-(C₁-C₆)-alkyl, CON(R8)(R9), N(R8)CO(R9), N(R8)SO₂(R9), CO(R8), (CR8R9)ₘ -O(R10), O-CO-N(R8)(R9), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R8)(R9);
m, m', m'' 0, 1, 2, 3, 4, 5, 6;
R5, R6, R7, R8, R9, R10 identically or differently hydrogen, (C₁-C₈)-alkyl;
or -(C=O)-NR1aR2a;
or -(C=O)-O-R1b;
or
R and X for X = -C(R3)(R4)- form a monocyclic, saturated or partly unsaturated 4- to 7-membered ring system or a bicyclic saturated or partly unsaturated 8- to 14-membered ring system whose individual members of the ring systems may be replaced by one to three atoms or atomic groups from the series -CHR11-, -CR11 R12-, -(C=R11)-, =C(R11)-, -NR11-,-C(=O)-, -O-, -S-, -SO-, -SO₂-, with the proviso that two units from the series -O-, -S-, -SO-, -SO₂- may not be adjacent;
R11, R12 identically or differently hydrogen, (C₁-C₆)-alkyl, aryl, (C₃-C₁₂)-cycloalkyl, (C₁-C₄)-alkylene-aryl, (C₁-C₃)-alkylene-(C₃-C₁₂)-cycloalkyl;
where aryl or cycloalkyl may be substituted by F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₂-C₆)-alkynyl, N(R13)(R14), SO₂-CH₃, SO₂-NH₂, SF₅, COOH, COO-(C₁-C₆)-alkyl, CON(R13)(R14), N(R13)CO(R14), N(R13)SO₂(R14), CO(R13), (CR13R14)ₙ-O(R15), O-CO-N(R13)(R14), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R13)(R14);
n 0, 1, 2, 3, 4, 5, 6;
R13, R14, R15 identically or differently hydrogen, (C₁-C₈)-alkyl;
R1, R1a, R1b identically or differently (C₅-C₁₆)-alkyl, CH₂-aryl, (C₁-C₂)-alkylene-heteroaryl, CH₂-(C₅-C₁₂)-cycloalkyl, where aryl, heteroaryl or cycloalkyl may be substituted one or more times by F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₃-C₈)-cycloalkyl, O-(C₃-C₈)-cycloalkyl, (C₂-C₆)-alkynyl, (C₀-C₈)-alkylene-aryl, O-(C₀-C₈)-alkylene-aryl, S-aryl, (C₀-C₈)-alkylene-heteroaryl, N(R16)(R17), SO₂-CH₃, SO₂-NH₂, SF₅, COOH, COO-(C₁-C₆)-alkyl, CON(R16)(R17), N(R16)CO(R17), N(R16)SO₂(R17), CO(R16), (CR16R17)ₒ-O(R18), O-CO-N(R16)(R17), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R16)(R17), where aryl or heteroaryl in turn may be substituted one or more times by
F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, (C₃-C₈)-cycloalkyl, O-(C₃-C₈)-cycloalkyl, (C₂-C₆)-alkynyl, N(R19)(R20), SO₂-CH₃, SF₅, COOH, COO-(C₁-C₆)-alkyl, CON(R19)(R20), N(R19)CO(R20), N(R19)SO₂(R20), CO(R19), (CR19R20)_{o'}-O(R21), O-CO-N(R19)(R20), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R19)(R20);
o, o' 0, 1, 2, 3, 4, 5, 6;
R16, R17, R18, R19, R20, R21 identically or differently hydrogen, (C₁-C₈)-alkyl;
or a radical of the formula la 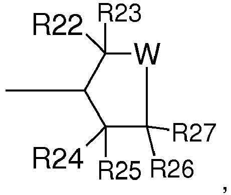 with
W -C(R28)(R29)-, -C(R28)(R29)-C(R28a)(R29a)-, C(R28)(R29)-O-;
R22, R23, R24, R25, R26, R27, R28, R29, R28a, R29a identically or differently hydrogen, F, Cl, Br, I, OH, CF₃, NO₂, CN, OCF₃, SF₅, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₃-C₈)-cycloalkyl, O-(C₃-C₈)-cycloalkyl, (C₃-C₈)-cycloalkenyl, (C₂-C₆)-alkynyl, N(R30)(R31), SO₂-CH₃, COOH, COO-(C₁-C₆)-alkyl, CON(R30)(R31), N(R30)CO(R31), N(R30)SO₂(R31), CO(R30), (CR30R31)ₚ-O(R32), O-CO-N(R30)(R31), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R30)(R31);
p 0, 1, 2, 3, 4, 5, 6;
R30, R31, R32 identically or differently hydrogen, (C₁-C₆)-alkyl;
or
R22 and R28 or R23 and R29 together with the carbon atoms bearing them form a monocyclic, 5 or 6 membered saturated, partly unsaturated or aromatic ring system whose individual members may be replaced by - CHR33-, -CR33R34-, =(C-R33)-;
or
R24 and R26, or R25 and R27 together with the carbon atoms bearing them form a monocyclic, 5 or 6 membered saturated, partly unsaturated or an aromatic ring system whose individual members may be replaced by - CHR33-, -CR33R34-, =(C-R33)-;
R33, R34 identically or differently F, Cl, Br, I, OH, CF₃, NO₂, CN, OCF₃, SF₅, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₃-C₈)-cycloalkyl, O-(C₃-C₈)-cycloalkyl, (C₂-C₆)-alkynyl, N(R35)(R36), SO₂-CH₃, COOH, COO-(C₁-C₆)-alkyl, CON(R35)(R36), N(R35)CO(R36), N(R35)SO₂(R36), CO(R35), (CR35R36)_{q}-O(R37), O-CO-N(R35)(R36), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R35)(R36);
q 0, 1, 2, 3, 4, 5, 6;
R35, R36, R37 identically or differently hydrogen, (C₁-C₆)-alkyl;
R2, R2a identically or differently hydrogen; (C₁-C₈)-alkyl;
R3, R4 identically or differently hydrogen, (C₁-C₆)-alkyl, benzyl;
the tautomeric forms of the compound and the physiologically tolerated salts thereof;
with the proviso that R1 is not pentyl, CH₂-phenyl, -CH₂-(2-Cl-phenyl), cyclohexyl, -(2-methylcyclohexyl) if X = CH₂, Y = CO, R = methyl, R2 = H.

2. A compound of the formula I as claimed in claim 1, in which R2 is hydrogen.

3. A compound of the formula I as claimed in claims 1 or 2, in which
Y is -(C=O)- and
X is -C(R3)(R4)-;
or
X is -(C=O)- and
Y is -C(R3(R4)-;
R is hydrogen, (C₁-C₃)-haloalkyl, (CR5R6)ₘ-O(R7), phenyl, heterocycle, (C₁-C₄)-alkylene-phenyl, (C₁-C₄)-alkylene-heteroaryl, (C₁-C₄)-alkylene-(C₅-C₁₂)-cycloalkyl, where cycloalkyl, phenyl, heterocycle or heteroaryl may be substituted one or more times by F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₃-C₈)-cycloalkyl, O-(C₃-C₈)-cycloalkyl, (C₂-C₆)-alkynyl, (C₀-C₈)-alkylene-aryl, O-(C₀-C₈)-alkylene-aryl, S-aryl, (C₀-C₈)-alkylene-heteroaryl, N(R5)(R6), SO₂-CH₃, SO₂-NH₂, SF₅, COOH, COO-(C₁-C₆)-alkyl, CON(R5)(R6), N(R5)CO(R6), N(R5)SO₂(R6), CO(R5), (CR5R6)ₘ -O(R7), O-CO-N(R5)(R6), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R5)(R6), where aryl or heteroaryl may in turn be substituted one or more times by
F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, (C₃-C₈)-cycloalkyl, O-(C₃-C₈)-cycloalkyl, (C₂-C₆)-alkynyl, N(R8)(R9), SO₂-CH₃, SF₅, COOH, COO-(C₁-C₆)-alkyl, CON(R8)(R9), N(R8)CO(R9), N(R8)SO₂(R9), CO(R8), (CR8R9)_{m''}-O(R10), O-CO-N(R8)(R9), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R8)(R9);
m, m', m'' are 0, 1, 2, 3, 4, 5, 6;
R5, R6, R7, R8, R9, R10 are identically or differently hydrogen, (C₁-C₈)-alkyl;
or -(C=O)-NR1aR2a;
or -(C=O)-O-R1b;
or
R and X for X = -C(R3)(R4)- form a monocyclic, saturated 5- to 7-membered ring system or a bicyclic partly unsaturated 8- to 14 membered ring system whose individual members may be replaced by one to three atoms or atomic groups from the series -CHR11-, -CR11R12-, -(C=R11)-, -NR11-, -C(=O)-,-O-, with the proviso that two units from the series -O- may not be adjacent;
R11, R12 are identically or differently hydrogen, (C₁-C₆)-alkyl, phenyl, (C₃-C₁₂)-cycloalkyl, (C₁-C₄)-alkylene-phenyl, (C₁-C₃)-alkylene-(C₃-C₁₂)-cycloalkyl; where phenyl or cycloalkyl may be substituted by F, Cl**,** Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₂-C₆)-alkynyl, N(R13)(R14), SO₂-CH₃, SO₂-NH₂, SF₅, COOH, COO-(C₁-C₆)-alkyl, CON(R13)(R14), N(R13)CO(R14), N(R13)SO₂(R14), CO(R13), (CR13R14)ₙ-O(R15), O-CO-N(R13)(R14), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R13)(R14);
n is 0, 1, 2, 3, 4, 5, 6;
R13, R14, R15 are independently of one another hydrogen, (C₁-C₈)-alkyl;
R1, R1 a, R1 b are identically or differently (C₅-C₁₂)-alkyl, -CH₂-phenyl, (C₁-C₂)-alkylene-heteroaryl, -CH₂-(C₅-C₁₂)-cycloalkyl, (C₅-C₆)-cycloalkyl, where phenyl, heteroaryl or cycloalkyl may be substituted one or more times by F, Cl, Br, I, OH, CF₃, N0₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₃-C₈)-cycloalkyl, O-(C₃-C₈)-cycloalkyl, (C₂-C₆)-alkynyl, (C₀-C₈)-alkylene-phenyl, O-(C₀-C₈)-alkylene-phenyl, S-phenyl, (C₀-C₈)-alkylene-heteroaryl, N(R16)(R17), SO₂-CH₃, SO₂-NH₂, SF₅, COOH, COO-(C₁-C₆)-alkyl, CON(R16)(R17), N(R16)CO(R17), N(R16)SO₂(R17), CO(R16), (CR16R17)ₒ-O(R18), O-CO-N(R16)(R17), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R16)(R17), where phenyl or heteroaryl may in turn be substituted one or more times by
F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, (C₂-C₆)-alkynyl, N(R19)(R20), SO₂-CH₃, SF₅, COOH, COO-(C₁-C₆)-alkyl, CON(R19)(R20), N(R19)CO(R20), N(R19)SO₂(R20), CO(R19), (CR19R20)_{o'}-O(R21), O-CO-N(R19)(R20), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R19)(R20);
o, o' are 0, 1, 2, 3, 4, 5, 6;
R16, R17, R18, R19, R20, R21 are identically or differently hydrogen, (C₁-C₈)-alkyl;
or a radical of the formula Ib

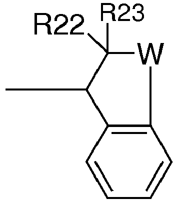

4. A compound of the formula I as claimed in claims 1 to 3, in which
Y is -(C=O)- and
X is -C(R3)(R4)-;
or
X is -(C=O)- and
Y is -C(R3(R4)-;
R is hydrogen, (CR5R6)ₘ-O(R7), phenyl, -CH₂-phenyl, where phenyl may be substituted once or twice by F, Cl, Br, CF₃, O-(C₁-C₆)-alkyl, (C₁-C₆)-alkyl, O-(C₂-C₄)-haloalkyl, N(R5)(R6), SO₂-CH₃, SO₂-NH₂, COOH, COO-(C₁-C₆)-alkyl, CON(R5)(R6), CO(R5), (CR5R6)_{m'}-O(R7),
m, m' are 0, 1, 2, 3;
R5, R6, R7 are identically or differently hydrogen, (C₁-C₈)-alkyl;
or
R and X for X = -C(R3)(R4)- form a monocyclic, saturated 6-membered ring system or a bicyclic partly unsaturated 9- to 11-membered ring system whose individual members may be replaced by one to two atoms or atomic groups from the series -CHR11-, -CR11 R12-, -(C=R11)-, =C(R11)-;
R11, R12 are identically or differently hydrogen, (C₁-C₆)-alkyl;
R1 is (C₅-C₈)-alkyl, -CH₂-phenyl, (C₁-C₂)-alkylene-heteroaryl, where heteroaryl is selected from the group of thiophene, benzothiophene, pyridine, pyrazole, -CH₂-(C₅-C₇)-cycloalkyl, (C₅-C₆)-cycloalkyl, where phenyl, heteroaryl or cycloalkyl may be substituted one or more times by F, Cl, Br, OH, CF₃, NO₂, CN, OCF₃, O-(C₁-C₆)-alkyl, (C₁-C₆)-alkyl, (C₀-C₈)-alkylene-phenyl, O-(C₀-C₈)-alkylene-phenyl, (C₀-C₈)-alkylene-heteroaryl, N(R16)(R17), SO₂-CH₃, SO₂-NH₂, COOH, COO-(C₁-C₆)-alkyl, CON(R16)(R17), CO(R16), (CR16R17)ₒ-O(R18), where phenyl or heteroaryl may in turn be substituted one or more times by
F, Cl, Br, OH, CF₃, NO₂, CN, OCF₃, O-(C₁-C₆)-alkyl, (C₁-C₆)-alkyl, COOH, COO-(C₁-C₆)-alkyl, CON(R19)(R20), CO(R19), (CR19R20)_{o'}-O(R21);
o, o' are 0, 1, 2, 3;
R16, R17, R18, R19, R20, R21 are identically or differently hydrogen, (C₁-C₈)-alkyl;
or a radical from the group 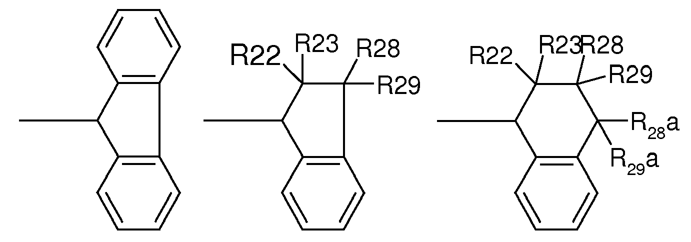 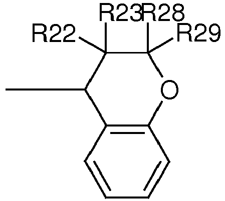 R22, R23, R28, R29, R28a, R29a are identically or differently hydrogen, (C₁-C₆)-alkyl, preferably hydrogen and methyl;
R2 is hydrogen;
R3, R4 are identically or differently hydrogen, methyl.

5. A compound of the formula I as claimed in claims 1 to 4, in which
Y is -(C=O)- and
X is -C(R3)(R4)-;
or
X is -(C=O)- and
Y is -C(R3(R4)-;
R is hydrogen, (CR5R6)ₘ-O(R7), -CH₂-phenyl, where phenyl may be substituted once or twice by F, Cl, Br, CF₃, O-(C₁-C₆)-alkyl, (C₁-C₆)-alkyl, O-(C₂-C₄)-haloalkyl, COOH, COO-(C₁-C₆)-alkyl, CON(R5)(R6), CO(R5), (CR5R6)_{m'}-O(R7),
m, m' are 0, 1, 2, 3;
R5, R6, R7 are identically or differently hydrogen, (C₁-C₄)-alkyl;
or
R and X for X = -C(R3)(R4)- form a monocyclic, saturated 6-membered ring system to which a benzene nucleus may be fused, whose individual members of the ring systems may be replaced by one to two atomic groups from the series-CHR11-, -CR11 R12-, -(C=R11)-, =C(R11)- ;
R11, R12 are identically or differently hydrogen, (C₁-C₆)-alkyl;
R1 is (C₅-C₈)-alkyl, -CH₂-phenyl, (C₁-C₂)-alkylene-heteroaryl, where heteroaryl is selected from the group of thiophene, benzothiophene, -CH₂-cyclohexyl, cyclohexyl, where phenyl, heteroaryl or cyclohexyl may be substituted once or twice by F, Cl, OH, CF₃, OCF₃, O-(C₁-C₆)-alkyl, (C₁-C₆)-alkyl, (C₀-C₁)-alkylene-phenyl, O-phenyl, (C₀-C₁)-alkylene-heteroaryl, N(R16)(R17), COOH, COO-(C₁-C₆)-alkyl, CON(R16)(R17), CO(R16), (CR16R17)ₒ-O(R18), where phenyl or heteroaryl may in turn be substituted once or twice by
F, Cl, OH, CF₃, O-(C₁-C₆)-alkyl, (C₁-C₆)-alkyl, COOH, COO-(C₁-C₆)-alkyl, CON(R19)(R20), CO(R19), (CR19R20)_{o'}-O(R21);
o, o' are 0, 1, 2, 3;
R16, R17, R18, R19, R20, R21 are identically or differently hydrogen, (C₁-C₈)-alkyl;
or a radical from the group 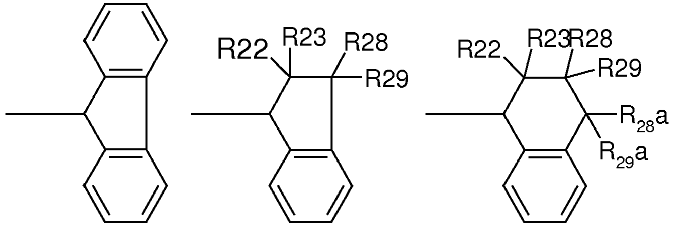 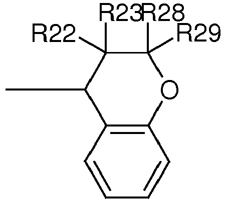 R22, R23, R28, R29, R28a, R29a are identically or differently hydrogen, (C₁-C₆)-alkyl, preferably hydrogen and methyl;
R2 is hydrogen;
R3, R4 are identically or differently hydrogen, methyl.

6. A compound of the formula I as claimed in claims 1 to 5, in which
Y is -(C=O)- and
X is -C(R3)(R4)-;
or
X is -(C=O)- and
Y is -C(R3)(R4)-;
R is hydrogen, HO-CH₂-, benzyl,
or
R and X for X = -C(-R3)(R4)- form -CH₂-CH₂-CH₂-CH₂- or 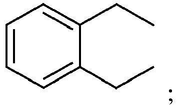
R1 is pentyl, hexyl, heptyl, cyclohexyl, -CH₂-cyclohexyl, -CH₂-phenyl, -CH₂-thiophene, -CH₂-CH₂-thiophene, where cyclohexyl, phenyl or thiophene may be substituted by methyl;
or a radical from the group 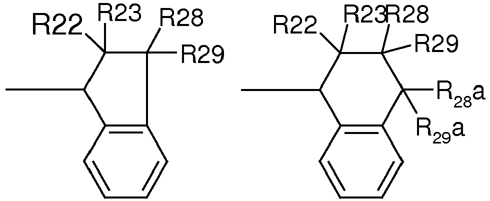
R22, R23, R28, R29, R28a, R29a are hydrogen;
R2 is hydrogen;
R3, R4 are identically or differently hydrogen, methyl.

7. A medicament comprising one or more compounds of the formula I as claimed in claims 1 to 6.

8. A medicament comprising one or more compounds of the formula I 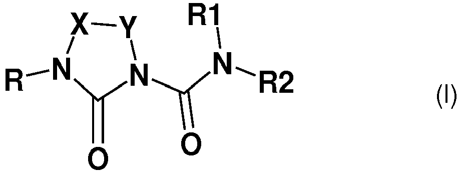 in which the meanings are:
X, Y identically or differently -C(R3)(R4)-, -(C=O)-, -(C=S), where at least one X or Y is -(C=O)- or -(C=S)-; but cannot both simultaneously be -(C=O)- or -(C=S)-; or X and Y together are C(R3)=C(R3);
R hydrogen, (C₁-C₈)-alkyl, (C₁-C₅)-haloalkyl, (CR5R6)ₘ-O(R7), (C₁-C₃)-alkyloxy-(C₁-C₃)-alkylene, aryl, heterocycle, (C₁-C₄)-alkylene-aryl, (C₁-C₄)-alkylene-heteroaryl, (C₁-C₄)-alkylene-(C₅-C₁₂)-cycloalkyl, where cycloalkyl, aryl, heterocycle or heteroaryl may be substituted one or more times by F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₃-C₈)-cycloalkyl, O-(C₃-C₈)-cycloalkyl, (C₂-C₆)-alkynyl, (C₀-C₈)-alkylene-aryl, O-(C₀-C₈)-alkylene-aryl, S-aryl, (C₀-C₈)-alkylene-heteroaryl, N(R5)(R6), SO₂-CH₃, SO₂-NH₂, SF₅, COOH, COO-(C₁-C₆)-alkyl, CON(R5)(R6), N(R5)CO(R6), N(R5)SO₂(R6), CO(R5), (CR5R6)_{m'}-O(R7), O-CO-N(R5)(R6), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R5)(R6), where aryl or heteroaryl may in turn be substituted one or more times by
F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, (C₃-C₈)-cycloalkyl, O-(C₃-C₈)-cycloalkyl, (C₂-C₆)-alkynyl, N(R8)(R9), SO₂-CH₃, SF₅, COOH, COO-(C₁-C₆)-alkyl, CON(R8)(R9), N(R8)CO(R9), N(R8)SO₂(R9), CO(R8), (CR8R9)_{m''}-O(R10), O-CO-N(R8)(R9), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R8)(R9);
m, m', m'' 0, 1, 2, 3, 4, 5, 6;
R5, R6, R7, R8, R9, R10 identically or differently hydrogen, (C₁-C₈)-alkyl;
or -(C=O)-NR1aR2a;
or -(C=O)-O-R1b;
or
R and X for X = -C(R3)(R4)- form a monocyclic, saturated or partly unsaturated 4- to 7-membered ring system or a bicyclic saturated or partly unsaturated 8- to 14-membered ring system whose individual members of the ring systems may be replaced by one to three atoms or atomic groups from the series -CHR11-, -CR11R12-, -(C=R11)-, =C(R11)-, -NR11-,-C(=O)-, -O-, -S-, -SO-, -SO₂-, with the proviso that two units from the series -O-, -S-, -SO-, -SO₂- may not be adjacent;
R11, R12 identically or differently hydrogen, (C₁-C₆)-alkyl, aryl, (C₃-C₁₂)-cycloalkyl, (C₁-C₄)-alkylene-aryl, (C₁-C₃)-alkylene-(C₃-C₁₂)-cycloalkyl;
where aryl or cycloalkyl may be substituted by F, Cl**,** Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₂-C₆)-alkynyl, N(R13)(R14), SO₂-CH₃, SO₂-NH₂, SF₅, COOH, COO-(C₁-C₆)-alkyl, CON(R13)(R14), N(R13)CO(R14), N(R13)SO₂(R14), CO(R13), (CR13R14)ₙ-O(R15), O-CO-N(R13)(R14), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R13)(R14);
n 0, 1, 2, 3, 4, 5, 6;
R13, R14, R15 identically or differently hydrogen, (C₁-C₈)-alkyl;
R1, R1a, R1b identically or differently (C₅-C₁₆)-alkyl, CH₂-aryl, (C₁-C₂)-alkylene-heteroaryl, CH₂-(C₅-C₁₂)-cycloalkyl, where aryl, heteroaryl or cycloalkyl may be substituted one or more times by F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₃-C₈)-cycloalkyl, O-(C₃-C₈)-cycloalkyl, (C₂-C₆)-alkynyl, (C₀-C₈)-alkylene-aryl, O-(C₀-C₈)-alkylene-aryl, S-aryl, (C₀-C₈)-alkylene-heteroaryl, N(R16)(R17), SO₂-CH₃, SO₂-NH₂, SF₅, COOH, COO-(C₁-C₆)-alkyl, CON(R16)(R17), N(R16)CO(R17), N(R16)SO₂(R17), CO(R16), (CR16R17)ₒ-O(R18), O-CO-N(R16)(R17), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R16)(R17), where aryl or heteroaryl in turn may be substituted one or more times by
F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, (C₃-C₈)-cycloalkyl, O-(C₃-C₈)-cycloalkyl, (C₂-C₆)-alkynyl, N(R19)(R20), SO₂-CH₃, SF₅, COOH, COO-(C₁-C₆)-alkyl, CON(R19)(R20), N(R19)CO(R20), N(R19)SO₂(R20), CO(R19), (CR19R20)_{o'}-O(R21), O-CO-N(R19)(R20), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R19)(R20);
o, o' 0, 1, 2, 3, 4, 5, 6;
R16, R17, R18, R19, R20, R21 identically or differently hydrogen, (C₁-C₈)-alkyl;
or a radical of the formula la 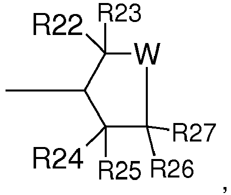 with
W -C(R28)(R29)-, -C(R28)(R29)-C(R28a)(R29a)-, C(R28)(R29)-O-;
R22, R23, R24, R25, R26, R27, R28, R29, R28a, R29a identically or differently hydrogen, F, Cl, Br, I, OH, CF₃, NO₂, CN, OCF₃, SF₅, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₃-C₈)-cycloalkyl, O-(C₃-C₈)-cycloalkyl, (C₃-C₈)-cycloalkenyl, (C₂-C₆)-alkynyl, N(R30)(R31), SO₂-CH₃, COOH, COO-(C₁-C₆)-alkyl, CON(R30)(R31), N(R30)CO(R31), N(R30)SO₂(R31), CO(R30), (CR30R31)ₚ-O(R32), O-CO-N(R30)(R31), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R30)(R31);
p 0, 1, 2, 3, 4, 5, 6;
R30, R31, R32 identically or differently hydrogen, (C₁-C₆)-alkyl;
or
R22 and R28 or R23 and R29 together with the carbon atoms bearing them form a monocyclic, 5 or 6 membered saturated, partly unsaturated or aromatic ring system whose individual members may be replaced by - CHR33-, -CR33R34-, =(C-R33)-;
or
R24 and R26, or R25 and R27 together with the carbon atoms bearing them form a monocyclic, 5 or 6 membered saturated, partly unsaturated or an aromatic ring system whose individual members may be replaced by - CHR33-, -CR33R34-, =(C-R33)-;
R33, R34 identically or differently F, Cl, Br, I, OH, CF₃, NO₂, CN, OCF₃, SF₅, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₃-C₈)-cycloalkyl, O-(C₃-C₈)-cycloalkyl, (C₂-C₆)-alkynyl, N(R35)(R36), SO₂-CH₃, COOH, COO-(C₁-C₆)-alkyl, CON(R35)(R36), N(R35)CO(R36), N(R35)SO₂(R36), CO(R35), (CR35R36)_{q}-O(R37), O-CO-N(R35)(R36), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R35)(R36);
q 0, 1, 2, 3, 4, 5, 6;
R35, R36, R37 identically or differently hydrogen, (C₁-C₆)-alkyl;
R2, R2a identically or differently hydrogen; (C₁-C₈)-alkyl;
R3, R4 identically or differently hydrogen, (C₁-C₆)-alkyl, benzyl;
the tautomeric forms of the compound and the physiologically tolerated salts thereof;
and which comprises as further active ingredient one or more antidiabetics, hypoglycemic active ingredients, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients acting on the ATP-dependent potassium channel of the beta cells, CART agonists, NPY agonists, MC4 agonists, orexin antagonists, H3 agonists, TNF agonists, CRF antagonists, CRF BP antagonists, urocortin agonists, β3 agonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

9. The use of the compounds of the formula I as claimed in claim 8 for the production of a medicament for the treatment and/or prevention of disorders of fatty acid metabolism and glucose utilization disorders.

10. The use of the compounds of the formula I as claimed in claim 8 for the production of a medicament for the treatment and/or prevention of dyslipidemias and the sequelae thereof.

11. The use of the compounds of the formula I as claimed in claim 8 for the production of a medicament for the treatment and/or prevention of conditions associated with the metabolic syndrome and disorders in which insulin resistance is involved, or for the treatment and/or prevention of diabetes mellitus and the sequelae associated therewith.

12. The use of the compounds of the formula I as claimed in claim 8 for the production of a medicament for the treatment and/or prevention of conditions associated with reduced HDL level.

13. The use of the compounds of the formula I as claimed in claim 8 for the production of a medicament for the treatment and/or prevention of atherosclerotic disorders.

14. The use of the compounds of the formula I as claimed in claim 8 in combination with at least one further active ingredient for the production of a medicament for the treatment and/or prevention of disorders in which insulin resistance is involved.

15. A process for producing a medicament comprising one or more of the compounds of the formula I as claimed in claim 8, which comprises mixing the latter with a pharmaceutically suitable carrier and converting this mixture into a form suitable for administration.

## Revendications

1. Composé de formule générale I 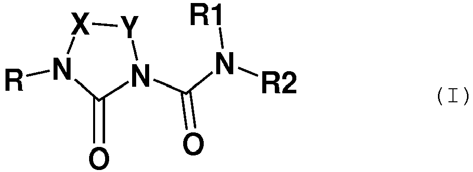 dans lequel:
X, Y sont, identiques ou différents, -C(R3)(R4)-, -(C=O)-, -(C=S), où au moins l'un de X ou Y est - (C=0) - ou - (C=S) - ; mais ils ne peuvent pas être simultanément -(C=0)- ou -(C=S)- ; ou X et Y sont conjointement C(R3)=C(R3) ;
R est un hydrogène, un halogénoalkyle en C₁-C₅, (CR5R6)ₘ-O(R7), (alkyloxy en C₁-C₃)-(alkylène en C₁-C₃), un aryle, un hétérocycle, (alkylène en C₁-C₄)-aryle, un (alkylène en C₁-C₄)-hétéroaryle, un (alkylène en C₁-C₄)-(cycloalkyle en C₅-C₁₂), où le cycloalkyle, l'aryle, l'hétérocycle ou l'hétéroaryle peuvent être substitués une ou plusieurs fois par F, Cl, Br, I, OH, CF₃, NO₂, CN, OCF₃, O-(alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un cycloalkyle en C₃-C₈, O-(cycloalkyle en C₃-C₈), un alcynyle en C₂-C₆, un (alkylène en C₀-C₈) -aryle, O-(alkylène en C₀-C₈) -aryle, S-aryle, un (alkylène en C₀-C₈)-hétéroaryle, N(R5)(R6), SO₂-CH₃, SO₂-NH₂, SF₅, COOH, COO-(alkyle en C₁-C₆), CON(R5)(R6), N(R5)CO(R6), N(R5)SO₂(R6), CO(R5), (CR5R6)_{m'}-O(R7), O-CO-N(R5)(R6), O-CO-(alkylène en C₁-C₆)-CO-O-(alkyle en C₁-C₆), O-CO-(alkylène en C₁-C₆-CO-OH, O-CO-(alkylène en C₁-C₆)-CO-N(R5)(R6), où l' aryle ou l'hétéroaryle peuvent eux-mêmes être substitués une ou plusieurs fois par
F, Cl, Br, I, OH, CF₃, NO₂, CN, OCF₃, O-(alkyle en C₁-C₆, O-(alcoxy en C₁-C₄)-(alkyle en C₁-C₄), S-(alkyle en C₁-C₆), un alkyle en C₁-C₆, un alcényle en C₂-C₆, un cycloalkyle en C₃-C₈, O-(cycloalkyle en C₃-C₈), un alcynyle en C₂-C₆, N(R8)(R9), SO₂-CH₃, SF₅, COOH, COO-(alkyle en C₁-C₆), CON(R8)(R9), N(R8)CO(R9), N(R8)SO₂(R9), CO(R8), (CR8R9)_{m''}-O(R10), O-CO-N(R8)(R9), O-CO- (alkylène en C₁-C₆-CO-O-(alkyle en C₁-C₆), O-CO-(alkylène en C₁-C₆)-CO-OH, O-CO-(alkylène en C₁-C₆)-CO-N(R8)(R9) ;
m, m', m'' sont 0, 1, 2, 3, 4, 5, 6 ;
R5, R6, R7, R8, R9, R10 sont, identiques ou différents, un hydrogène, un alkyle en C₁-C₈ ;
ou -(C=O)-NR1aR2a ;
ou -(C=O)-O-R1b ;
ou
R et X pour X = -C(R3)(R4)- forment un système cyclique monocyclique de 4 à 7 chaînons saturé ou partiellement insaturé ou un système cyclique bicyclique de 8 à 14 chaînons saturé ou partiellement insaturé dont les chaînons individuels des systèmes cycliques peuvent être remplacés par un à trois atomes ou groupes atomiques de la série -CHR11-, -CR11R12-, -(C=R11)-, =C(R11)-, -NR11-, -C(=O)-, -0-, -S-, -SO-, -SO₂-, à condition que deux motifs de la série -O-,-S-, -SO-, -SO₂- ne soient pas adjacents ;
R11, R12 sont, identiques ou différents, un hydrogène, un alkyle en C₁-C₆, un aryle, un cycloalkyle en C₃-C₁₂, un (alkylène en C₁-C₄)-aryle, un (alkylène en C₁-C₃)-(cycloalkyle en C₃-C₁₂) ; où l'aryle ou le cycloalkyle peuvent être substitués par F, Cl, Br, I, OH, CF₃, NO₂, CN, OCF₃, O- (alkyle en C₁-C₆), O- (alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un alcynyle en C₂-C₆, N(R13)(R14), SO₂-CH₃, SO₂-NH₂, SF₅, COOH, COO-(alkyle en C₁-C₆), CON(R13)(R14), N(R13)CO(R14), N(R13)SO₂(R14), CO(R13), (CR13R14)ₙ-0(R15), O-CO-N(R13)(R14), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R13)(R14) ;
n est 0, 1, 2, 3, 4, 5, 6 ;
R13, R14, R15 sont, identiques ou différents, un hydrogène, un alkyle en C₁-C₈ ;
R1, R1a, R1b sont, identiques ou différents, un alkyle en C₅-C₁₆, CH₂-aryle, un (alkylène en C₁-C₂)-hétéroaryle, CH₂-(cycloalkyle en C₅-C₁₂), où l'aryle, l'hétéroaryle ou le cycloalkyle peuvent être substitués une ou plusieurs fois par F, Cl, Br, I, OH, CF₃, NO₂, CN, OCF₃, O-(alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un cycloalkyle en C₃-C₈, O-(cycloalkyle en C₃-C₈), un alcynyle en C₂-C₆, un (alkylène en C₀-C₈)-aryle, O-(alkylène en C₀-C₈)-aryle, S-aryle, un (alkylène en C₀-C₈)-hétéroaryle, N(R16)(R17), SO₂-CH₃, SO₂-NH₂, SF₅, COOH, COO-(alkyle en C₁-C₆), CON(R16) (R17), N(R16)CO(R17), N(R16)SO₂(R17), CO(R16), (CR16R17)ₒ-O(R18), O-CO-N(R16)(R17), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R16)(R17), où l'aryle ou l'hétéroaryle peuvent eux-mêmes être substitués une ou plusieurs fois par
F, Cl, Br, I, OH, CF₃, NO₂, CN, OCF₃, O-(alkyle en C₁-C₆), O-(alcoxy en C₁-C₄)-(alkyle en C₁-C₄), S-(alkyle en C₁-C₆), un alkyle en C₁-C₆, un alcényle en C₂-C₆, un cycloalkyle en C₃-C₈, O-(cycloalkyle en C₃-C₈), un alcynyle en C₂-C₆, N(R19)(R20), SO₂-CH₃, SF₅, COOH, COO-(alkyle en C₁-C₆), CON(R19)(R20), N(R19)CO(R20), N(R19)SO₂(R20), CO(R19), (CR19R20)_{o'}-O(R21), O-CO-N(R19) (R20), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R19)(R20) ;
o, o´ sont 0, 1, 2, 3, 4, 5, 6 ;
R16, R17, R18, R19, R20, R21 sont, identiques ou différents, un hydrogène, un alkyle en C₁-C₈ ;
ou un radical de formule Ia 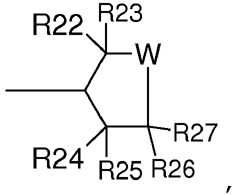 avec
W -C(R28)(R29)-, -C(R28)(R29)-C(R28a)(R29a)-, -C(R28)(R29)-O- ;
R22, R23, R24, R25, R26, R27, R28, R29, R28a, R29a sont, identiques ou différents, un hydrogène, F, Cl, Br, I, OH, CF₃, NO₂, CN, OCF₃, SF₅, 0- (alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un cycloalkyle en C₃-C₈, 0-(cycloalkyle en C₃-C₈), un cycloalcényle en C₃-C₈, un alcynyle en C₂-C₆, N(R30)(R31), SO₂-CH₃, COOH, COO-(alkyle en C₁-C₆), CON(R30)(R31), N(R30)CO(R31), N(R30)SO₂(R31), CO(R30), (CR30R31)ₚ-O(R32), O-CO-N(R30)(R31), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R30) (R31) ;
p est 0, 1, 2, 3, 4, 5, 6 ;
R30, R31, R32 sont, identiques ou différents, un hydrogène, un alkyle en C₁-C₆ ;
ou
R22 et R28 ou R23 et R29 conjointement avec les atomes de carbone portant ceux-ci forment un système cyclique monocyclique de 5 ou 6 chaînons, saturé, partiellement insaturé ou aromatique dont les chaînons individuels peuvent être substitués par -CHR33-, -CR33R34-, =(C-R33)- ;
ou
R24 et R26, ou R25 et R27 conjointement avec les atomes de carbone portant ceux-ci forment un système cyclique monocyclique de 5 ou 6 chaînons, saturé, partiellement insaturé ou aromatique dont les chaînons individuels peuvent être substitués par -CHR33-, -CR33R34-, =(C-R33)- ;
R33, R34 sont, identiques ou différents, F, Cl, Br, I, OH, CF₃, NO₂, CN, OCF₃, SF₅, O-(alkyle en C₁-C₆), 0-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un cycloalkyle en C₃-C₈, O-(cycloalkyle en C₃-C₈), un alcynyle en C₂-C₆, N(R35)(R36), SO₂-CH₃, COOH, COO-(alkyle en C₁-C₆), CON(R35)(R36), N(R35)CO(R36), N(R35)SO₂(R36), CO(R35), (CR35R36)_{q}-O(R37), O-CO-N(R35)(R36), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R35)(R36) ;
q est 0, 1, 2, 3, 4, 5, 6 ;
R35, R36, R37 sont, identiques ou différents, un hydrogène, un alkyle en C₁-C₆ ;
R2, R2a sont, identiques ou différents, un hydrogène, un alkyle en C₁-C₈ ;
R3, R4 sont, identiques ou différents, un hydrogène, un alkyle en C₁-C₆, un benzyle ;
les formes tautomères du composé et les sels physiologiquement tolérés de celui-ci ;
à condition que R1 ne soit pas un pentyle, CH₂-phényle, -CH₂-(2-Cl-phényle), un cyclohexyle, -(2-méthylcyclohexyle) si X = CH₂, Y = CO, R = méthyle, R2 = H.

2. Composé de formule I selon la revendication 1, dans lequel R2 est un hydrogène.

3. Composé de formule I selon les revendications 1 ou 2, dans lequel
Y est -(C=O)- et
X est -C (R3) (R4) - ;
ou
X est -(C=O)- et
Y est -C(R3(R4)- ;
R est un hydrogène, un halogénoalkyle en C₁-C₃, (CR5R6)ₘ-O(R7), un phényle, un hétérocycle, un (alkylène en C₁-C₄)-phényle, un (alkylène en C₁-C₄)-hétéroaryle, un (alkylène en C₁-C₄)-(cycloalkyle en C₅-C₁₂), où le cycloalkyle, le phényle, l'hétérocycle ou l'hétéroaryle peuvent être substitués une ou plusieurs fois par F, Cl, Br, I, OH, CF₃, NO₂, CN, OCF₃, O-(alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un cycloalkyle en C₃-C₈, O-(cycloalkyle en C₃-C₈), un alcynyle en C₂-C₆, un (alkylène en C₀-C₈)-aryle, O-(alkylène en C₀-C₈)-aryle, S-aryle, un (alkylène en C₀-C₈)-hétéroaryle, N(R5)(R6), SO₂-CH₃, SO₂-NH₂, SF₅, COOH, COO-(alkyle en C₁-C₆), CON(R5)(R6), N(R5)CO(R6), N(R5)SO₂(R6), CO(R5), (CR5R6)_{m'}-O(R7), O-CO-N(R5)(R6), O-CO-(alkylène en C₁-C₆)-CO-O-(alkyle en C₁-C₆), O-CO-(alkylène en C₁-C₆)-CO-OH, O-CO-(alkylène en C₁-C₆) -CO-N(R5) (R6), où l'aryle ou l'hétéroaryle peuvent eux-mêmes être substitués une ou plusieurs fois par
F, Cl, Br, I, OH, CF₃, NO₂, CN, OCF₃, O-(alkyle en C₁-C₆), O-(alcoxy en C₁-C₄)-(alkyle en C₁-C₄), S-(alkyle en C₁-C₆), un alkyle en C₁-C₆, un alcényle en C₂-C₆, un cycloalkyle en C₃-C₈, O-(cycloalkyle en C₃-C₈), un alcynyle en C₂-C₆, N(R8)(R9), SO₂-CH₃, SF₅, COOH, COO-(alkyle en C₁-C₆), CON(R8)(R9), N(R8)CO(R9), N(R8)SO₂(R9), CO(R8), (CR8R9)_{m''-}O(R10), O-CO-N(R8)(R9), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R8)(R9) ;
m, m', m'' sont 0, 1, 2, 3, 4, 5, 6 ;
R5, R6, R7, R8, R9, R10 sont, identiques ou différents, un hydrogène, un alkyle en C₁-C₈ ;
ou -(C=O)-NR1aR2a ;
ou -(C=O)-O-R1b ;
ou
R et X pour X = -C(R3)(R4)- forment un système cyclique monocyclique de 5 à 7 chaînons saturé ou un système cyclique bicyclique de 8 à 14 chaînons partiellement insaturé dont les chaînons individuels peuvent être remplacés par un à trois atomes ou groupes atomiques de la série -CHR11-, -CR11R12-, -(C=R11)-, -NR11-, -C(=O)-, -0-, à condition que deux motifs de la série -0- ne soient pas adjacents ;
R11, R12 sont, identiques ou différents, un hydrogène, un alkyle en C₁-C₆, un phényle, un cycloalkyle en C₃-C₁₂, un (alkylène en C₁-C₄)-phényle, un (alkylène en C₁-C₃)-(cycloalkyle en C₃-C₁₂) ; où le phényle ou le cycloalkyle peuvent être substitués par F, Cl, Br, I, OH, CF₃, NO₂, CN, OCF₃, O-(alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un alcynyle en C₂-C₆, N(R13)(R14), SO₂-CH₃, SO₂-NH₂, SF₅, COOH, COO-(alkyle en C₁-C₆), CON(R13)(R14), N(R13)CO(R14), N(R13)SO₂(R14), CO(R13), (CR13R14)ₙ-0(R15), O-CO-N(R13)(R14), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R13)(R14) ;
n est 0, 1, 2, 3, 4, 5, 6 ;
R13, R14, R15 sont, indépendamment les uns des autres, un hydrogène, un alkyle en C₁-C₈ ;
R1, R1a, R1b sont, identiques ou différents, un alkyle en C₅-C₁₂, -CH₂-phényle, un (alkylène en C₁-C₂) -hétéroaryle, -CH₂-(cycloalkyle en C₅-C₁₂), un cycloalkyle en C₅-C₆, où le phényle, l'hétéroaryle ou le cycloalkyle peuvent être substitués une ou plusieurs fois par F, Cl, Br, I, OH, CF₃, NO₂, CN, OCF₃, O-(alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un cycloalkyle en C₃-C₈, O-(cycloalkyle en C₃-C₈), un alcynyle en C₂-C₆, un (alkylène en C₀-C₈) -phényle, 0- (alkylène en C₀-C₈)-phényle, S-phényle, un (alkylène en C₀-C₈)-hétéroaryle, N(R16)(R17), SO₂-CH₃, SO₂-NH₂, SF₅, COOH, COO-(alkyle en C₁-C₆), CON(R16)(R17), N(R16)CO(R17), N(R16)SO₂(R17), CO(R16), (CR16R17)ₒ-O(R18), O-CO-N(R16)(R17), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R16)(R17), où le phényle ou l'hétéroaryle peuvent eux-mêmes être substitués une ou plusieurs fois par
F, Cl, Br, I, OH, CF₃, NO₂, CN, OCF₃, O(alkyle en C₁-C₆), O-(alcoxy en C₁-C₄)-(alkyle en C₁-C₄), S-(alkyle en C₁-C₆), un alkyle en C₁-C₆, un alcényle en C₂-C₆, un alcynyle en C₂-C₆, N(R19)(R20), SO₂-CH₃, SF₅, COOH, COO-(alkyle en C₁-C₆), CON(R19)(R20), N(R19)CO(R20), N(R19)SO₂(R20), CO(R19), (CR19R20)_{o'}-O(R21), O-CO-N(R19)(R20), O-CO-(alkylène en C₁-C₆)-CO-O- (alkyle en C₁-C₆), O-CO-(alkylène en C₁-C₆)-CO-OH, O-CO-(alkylène en C₁-C₆)-CO-N(R19)(R20) ;
o, o' sont 0, 1, 2, 3, 4, 5, 6 ;
R16, R17, R18, R19, R20, R21 sont, identiques ou différents, un hydrogène, un alkyle en C₁-C₈ ;
ou un radical de formule Ib

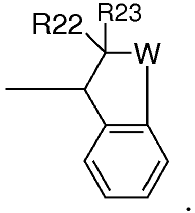

4. Composé de formule I selon les revendications 1 à 3, dans lequel
Y est -(C=O)- et
X est -C (R3) (R4) - ;
ou
X est -(C=O)- et
Y est -C(R3(R4)- ;
R est un hydrogène, (CR5R6)ₘ-O(R7), un phényle, -CH₂-phényle, où le phényle peut être substitué une ou deux fois par F, Cl, Br, CF₃, O-(alkyle en C₁-C₆), un alkyle en C₁-C₆, 0- (halogénoalkyle en C₂-C₄), N(R5)(R6), SO₂-CH₃, SO₂-NH₂, COOH, COO-(alkyle en C₁-C₆), CON(R5)(R6), CO(R5), (CR5R6)_{m'}-O(R7),
m, m' sont 0, 1, 2, 3 ;
R5, R6, R7 sont, identiques ou différents, un hydrogène, un alkyle en C₁-C₈ ;
ou
R et X pour X = -C(R3)(R4)- forment un système cyclique monocyclique de 6 chaînons saturé ou un système cyclique bicyclique de 9 à 11 chaînons partiellement insaturé dont les membres individuels peuvent être remplacés par un à deux atomes ou groupes atomiques de la série -CHR11-, -CR11R12-, -(C=R11)-, =C(R11)- ;
R11, R12 sont, identiques ou différents, un hydrogène, un alkyle en C₁-C₆ ;
R1 est un alkyle en C₅-C₈, -CH₂-phényle, un (alkylène en C₁-C₂)-hétéroaryle, où l'hétéroaryle est choisi dans le groupe comprenant le thiophène, le benzothiophène, la pyridine, le pyrazole, -CH₂-(cycloalkyle en C₅-C₇) un cycloalkyle en C₅-C₆, où le phényle, l'hétéroaryle ou le cycloalkyle peuvent être substitués une ou plusieurs fois par F, Cl, Br, OH, CF₃, NO₂, CN, OCF₃, O-(alkyle en C₁-C₆), un alkyle en C₁-C₆, un (alkylène en C₀-C₈)-phényle, O-(alkylène en C₀-C₈)-phényle, un (alkylène en C₀-C₈)-hétéroaryle, N(R16)(R17), SO₂-CH₃, SO₂-NH₂, COOH, COO-(alkyle en C₁-C₆), CON(R16)(R17), CO(R16), (CR16R17)ₒ-O(R18), où le phényle ou l'hétéroaryle peuvent eux-mêmes être substitués une ou plusieurs fois par
F, Cl, Br, OH, CF₃, NO₂, CN, OCF₃, O-(alkyle en C₁-C₆), un alkyle en C₁-C₆, COOH, COO-(alkyle en C₁-C₆), CON(R19)(R20), CO(R19), (CR19R20)_{o'}-O(R21) ;
o, o' sont 0, 1, 2, 3 ;
R16, R17, R18, R19, R20, R21 sont, identiques ou différents, un hydrogène, un alkyle en C₁-C₈ ;
ou un radical du groupe 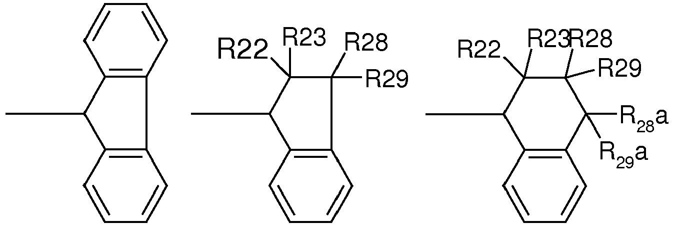 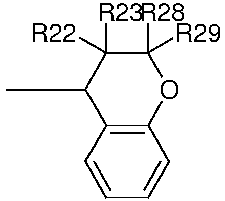 R22, R23, R28, R29, R28a, R29a sont, identiques ou différents, un hydrogène, un alkyle en C₁-C₆, de préférence un hydrogène et un méthyle ;
R2 est un hydrogène ;
R3, R4 sont, identiques ou différents, un hydrogène, un méthyle.

5. Composé de formule I selon les revendications 1 à 4, dans lequel
Y est -(C=O)- et
X est -C (R3) (R4) - ;
ou
X est -(C=O)- et
Y est -C(R3(R4)- ;
R est un hydrogène, (CR5R6)ₘ-O(R7), -CH₂-phényle, où le phényle peut être substitué une ou deux fois par F, Cl, Br, CF₃, O-(alkyle en C₁-C₆), un alkyle en C₁-C₆, O-(halogénoalkyle en C₂-C₄), COOH, COO-(alkyle en C₁-C₆), CON(R5)(R6), CO(R5), (CR5R6)_{m'}-O(R7),
m, m' sont 0, 1, 2, 3 ;
R5, R6, R7 sont, identiques ou différents, un hydrogène, un alkyle en C₁-C₄ ;
ou
R et X pour X = -C(R3)(R4)- forment un système cyclique monocyclique de 6 chaînons saturé auquel un noyau benzénique peut être condensé, dont les chaînons individuels des systèmes cycliques peuvent être remplacés par un à deux groupes atomiques de la série-CHR11-, -CR11R12-, -(C=R11)-, =C(R11)- ;
R11, R12 sont, identiques ou différents, un hydrogène, un alkyle en C₁-C₆ ;
R1 est un alkyle en C₅-C₈, -CH₂-phényle, un (alkylène en C₁-C₂)-hétéroaryle, où l'hétéroaryle est choisi dans le groupe comprenant le thiophène, le benzothiophène, -CH₂-cyclohexyle, un cyclohexyle, où le phényle, l'hétéroaryle ou le cyclohexyle peuvent être substitués une ou deux fois par F, Cl, OH, CF₃, OCF₃, O-(alkyle en C₁-C₆), un alkyle en C₁-C₆, (C₀-C₁) -alkylène-phényle, O-phényle, (C₀-C₁)-alkylène-hétéroaryle, N(R16)(R17), COOH, COO-(alkyle en C₁-C₆), CON(R16)(R17), CO(R16), (CR16R17)ₒ-O(R18), où le phényle ou l'hétéroaryle peuvent eux-mêmes être substitués une ou deux fois par
F, Cl, OH, CF₃, O-(alkyle en C₁-C₆), un alkyle en C₁-C₆, COOH, COO-(alkyle en C₁-C₆), CON(R19)(R20), CO(R19), (CR19R20)_{o'}-O(R21) ;
o, o' sont 0, 1, 2, 3 ;
R16, R17, R18, R19, R20, R21 sont, identiques ou différents, un hydrogène, un alkyle en C₁-C₈ ;
ou un radical du groupe 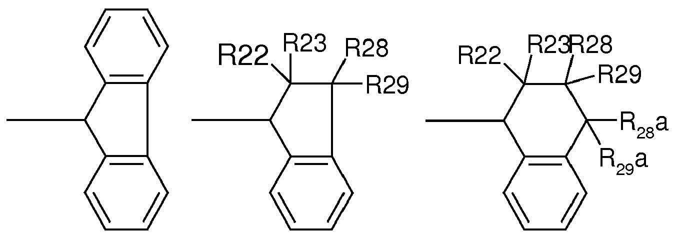 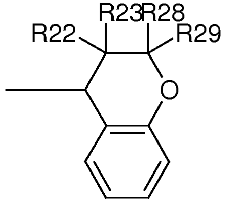 R22, R23, R28, R29, R28a, R29a sont, identiques ou différents, un hydrogène, un alkyle en C₁-C₆, de préférence un hydrogène et un méthyle ;
R2 est un hydrogène ;
R3, R4 sont, identiques ou différents, un hydrogène, un méthyle.

6. Composé de formule I selon les revendications 1 à 5, dans lequel
Y est -(C=O)- et
X est -C (R3) (R4) - ;
ou
X est -(C=O)- et
Y est -C (R3) (R4) - ;
R est un hydrogène, HO-CH₂-, un benzyle,
ou
R et X pour X = -C (-R3) (R4) - forment -CH₂-CH₂-CH₂-CH₂- ou 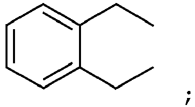
R1 est un pentyle, un hexyle, un heptyle, un cyclohexyle, -CH₂-cyclohexyle, -CH₂-phényle, -CH₂-thiophène, -CH₂-CH₂-thiophène, où le cyclohexyle, le phényle ou le thiophène peuvent être substitués par un méthyle ;
ou un radical du groupe 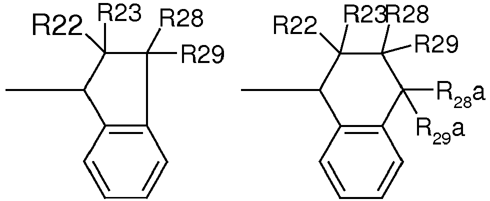
R22, R23, R28, R29, R28a, R29a sont un hydrogène ;
R2 est un hydrogène ;
R3, R4 sont, identiques ou différents, un hydrogène, un méthyle.

7. Médicament comprenant un ou plusieurs composés de formule I selon les revendications 1 à 6.

8. Médicament comprenant un ou plusieurs composés de formule I 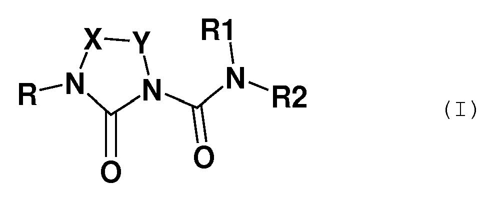
X, Y sont, identiques ou différents, -C(R3)(R4)-, -(C=O)-, -(C=S), où au moins l'un de X ou Y est -(C=O)- ou -(C=S)- ; mais ils ne peuvent pas être simultanément -(C=0)- ou -(C=S)- ; ou X et Y sont conjointement C(R3)=C(R3) ;
R est un hydrogène, un halogénoalkyle en C₁-C₅, (CR5R6)ₘ-O(R7), (alkyloxy en C₁-C₃)-(alkylène en C₁-C₃), un aryle, un hétérocycle, (alkylène en C₁-C₄)-aryle, un (alkylène en C₁-C₄)-hétéroaryle, un (alkylène en C₁-C₄)-(cycloalkyle en C₅-C₁₂), où le cycloalkyle, l'aryle, l'hétérocycle ou l'hétéroaryle peuvent être substitués une ou plusieurs fois par F, Cl, Br, I, OH, CF₃, NO₂, CN, OCF₃, O-(alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un cycloalkyle en C₃-C₈, O-(cycloalkyle en C₃-C₈), un alcynyle en C₂-C₆, un (alkylène en C₀-C₈)-aryle, O-(alkylène en C₀-C₈)-aryle, S-aryle, un (alkylène en C₀-C₈)-hétéroaryle, N(R5)(R6), SO₂-CH₃, SO₂-NH₂, SF₅, COOH, COO-(alkyle en C₁-C₆), CON(R5)(R6), N(R5)CO(R6), N(R5)SO2(R6), CO(R5), (CR5R6)_{m'}-O(R7), O-CO-N(R5)(R6), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R5) (R6), où l'aryle ou l'hétéroaryle peuvent eux-mêmes être substitués une ou plusieurs fois par
F, Cl, Br, I, OH, CF₃, NO₂, CN, OCF₃, O-(alkyle en C₁-C₆), O-(alcoxy en C₁-C₄)-(alkyle en C₁-C₄), S-(alkyle en C₁-C₆), un alkyle en C₁-C₆, un alcényle en C₂-C₆, un cycloalkyle en C₃-C₈, O-(cycloalkyle en C₃-C₈), un alcynyle en C₂-C₆, N(R8)(R9), SO₂-CH₃, SF₅, COOH, COO-(alkyle en C₁-C₆), CON(R8)(R9), N(R8)CO(R9), N(R8)SO₂(R9), CO(R8), (CR8R9)_{m''}-O(R10), O-CO-N(R8)(R9), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R8)(R9) ;
m, m', m'' sont 0, 1, 2, 3, 4, 5, 6 ;
R5, R6, R7, R8, R9, R10 sont, identiques ou différents, un hydrogène, un alkyle en C₁-C₈ ;
ou -(C=O)-NR1aR2a ;
ou -(C=O)-O-R1b ;
ou
R et X pour X = -C(R3)(R4)- forment un système cyclique monocyclique de 4 à 7 chaînons saturé ou partiellement insaturé ou un système cyclique bicyclique de 8 à 14 chaînons saturé ou partiellement insaturé dont les chaînons individuels des systèmes cycliques peuvent être remplacés par un à trois atomes ou groupes atomiques de la série -CHR11-, -CR11R12-, -(C=R11)-, =C(R11)-, -NR11-, -C(=O)-, -0-, -S-, -SO-, -SO₂-, à condition que deux motifs de la série -O-,-S-, -SO-, -SO₂- ne soient pas adjacents ;
R11, R12 sont, identiques ou différents, un hydrogène, un alkyle en C₁-C₆, un aryle, un cycloalkyle en C₃-C₁₂, un (alkylène en C₁-C₄)-aryle, un (alkylène en C₁-C₃)-(cycloalkyle en C₃-C₁₂) ; où l'aryle ou le cycloalkyle peuvent être substitués par F, Cl, Br, I, OH, CF₃, NO₂, CN, OCF₃, O-(alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un alcynyle en C₂-C₆, N(R13)(R14), SO₂-CH₃, SO₂-NH₂, SF₅, COOH, COO-(alkyle en C₁-C₆), CON(R13)(R14), N(R13)CO(R14), N(R13)SO₂(R14), CO(R13), (CR13R14)ₙ-0(R15), O-CO-N(R13)(R14), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R13)(R14) ;
n est 0, 1, 2, 3, 4, 5, 6 ;
R13, R14, R15 sont, identiques ou différents, un hydrogène, un alkyle en C₁-C₈ ;
R1, R1a, R1b sont, identiques ou différents, un alkyle en C₅-C₁₆, CH₂-aryle, un (alkylène en C₁-C₂)-hétéroaryle, CH₂-(cycloalkyle en C₅-C₁₂), où l'aryle, l'hétéroaryle ou le cycloalkyle peuvent être substitués une ou plusieurs fois par F, Cl, Br, I, OH, CF₃, NO₂, CN, OCF₃, O-(alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un cycloalkyle en C₃-C₈, O-(cycloalkyle en C₃-C₈), un alcynyle en C₂-C₆, un (alkylène en C₀-C₈)-aryle, O-(alkylène en C₀-C₈)-aryle, S-aryle, un (alkylène en C₀-C₈)-hétéroaryle, N(R16)(R17), SO₂-CH₃, SO₂-NH₂, SF₅, COOH, COO-(alkyle en C₁-C₆), CON(R16)(R17), N(R16)CO(R17), N(R16)SO₂(R17), CO(R16), (CR16R17)ₒ-O(R18), O-CO-N(R16)(R17), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R16)(R17), où l'aryle ou l'hétéroaryle peuvent eux-mêmes être substitués une ou plusieurs fois par
F, Cl, Br, I, OH, CF₃, NO₂, CN, OCF₃, O-(alkyle en C₁-C₆), O-(alcoxy en C₁-C₄)-(alkyle en C₁-C₄), S-(alkyle en C₁-C₆), un alkyle en C₁-C₆, un alcényle en C₂-C₆, un cycloalkyle en C₃-C₆, O-(cycloalkyle en C₃-C₈), un alcynyle en C₂-C₆, N(R19)(R20), SO₂-CH₃, SF₅, COOH, COO-(alkyle en C₁-C₆), CON(R19)(R20), N(R19)CO(R20), N(R19)SO₂(R20), CO(R19), (CR19R20)_{o'}-O(R21), O-CO-N(R19)(R20), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R19)(R20) ;
o, o' sont 0, 1, 2, 3, 4, 5, 6 ;
R16, R17, R18, R19, R20, R21 sont, identiques ou différents, un hydrogène, un alkyle en C₁-C₈ ;
ou un radical de formule Ia 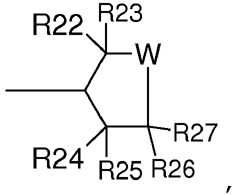 avec
W -C(R28)(R29)-, -C(R28)(R29)-C(R28a)(R29a)-, -C(R28)(R29)-O- ;
R22, R23, R24, R25, R26, R27, R28, R29, R28a, R29a sont, identiques ou différents, un hydrogène, F, Cl, Br, I, OH, CF₃, NO₂, CN, OCF₃, SF₅, O-(alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un cycloalkyle en C₃-C₈, O-(cycloalkyle en C₃-C₈), un cycloalcényle en C₃-C₈, un alcynyle en C₂-C₆, N(R30)(R31), SO₂-CH₃, COOH, COO-(alkyle en C₁-C₆), CON(R30)(R31), N(R30)CO(R31), N(R30)SO₂(R31), CO(R30), (CR30R31)ₚ-O(R32), O-CO-N(R30)(R31), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R30)(R31) ;
p est 0, 1, 2, 3, 4, 5, 6 ;
R30, R31, R32 sont, identiques ou différents, un hydrogène, un alkyle en C₁-C₆ ;
ou
R22 et R28 ou R23 et R29 conjointement avec les atomes de carbone portant ceux-ci forment un système cyclique monocyclique de 5 ou 6 chaînons, saturé, partiellement insaturé ou aromatique dont les chaînons individuels peuvent être substitués par -CHR33-, -CR33R34-, =(C-R33)- ;
ou
R24 et R26, ou R25 et R27 conjointement avec les atomes de carbone portant ceux-ci forment un système cyclique monocyclique de 5 ou 6 chaînons, saturé, partiellement insaturé ou aromatique dont les chaînons individuels peuvent être substitués par -CHR33-, -CR33R34-, =(C-R33)- ;
R33, R34 sont, identiques ou différents, F, Cl, Br, I, OH, CF₃, NO₂, CN, OCF₃, SF₅, O-(alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un cycloalkyle en C₃-C₈, O-(cycloalkyle en C₃-C₈), un alcynyle en C₂-C₆, N(R35)(R36), SO₂-CH₃, COOH, COO-(alkyle en C₁-C₆), CON(R35)(R36), N(R35)CO(R36), N(R35)SO₂(R36), CO(R35), (CR35R36)_{q}-O(R37), O-CO-N(R35)(R36), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R35)(R36) ;
q est 0, 1, 2, 3, 4, 5, 6 ;
R35, R36, R37 sont, identiques ou différents, un hydrogène, un alkyle en C₁-C₆ ;
R2, R2a sont, identiques ou différents, un hydrogène, un alkyle en C₁-C₈ ;
R3, R4 sont, identiques ou différents, un hydrogène, un alkyle en C₁-C₆, un benzyle ;
les formes tautomères du composé et les sels physiologiquement tolérés de celui-ci ;
et en tant que principe actif supplémentaire un ou plusieurs
antidiabétiques, principes actifs hypoglycémiques, inhibiteurs de HMGCoA réductase, inhibiteurs d'absorption de cholestérol, agonistes de PPAR gamma, agonistes de PPAR alpha, agonistes de PPAR alpha/gamma, fibrates, inhibiteurs de MTP, inhibiteurs d'absorption d'acide biliaire, inhibiteurs de CETP, adsorbants d'acide biliaire polymère, inducteurs de récepteur de LDL, inhibiteurs d'ACAT, antioxydants, inhibiteurs de lipoprotéine lipase, inhibiteurs d'ATP citrate lyase, inhibiteurs de squalène synthétase, antagonistes de lipoprotéine (a), inhibiteurs de lipase, insulines, sulfonylurées, biguanides, méglitinides, thiazolidinediones, inhibiteurs de α-glucosidase, principes actifs agissant sur le canal potassique ATP-dépendant des cellules bêta, agonistes de CART, agonistes de NPY, agonistes de MC4, antagonistes d'orexine, agonistes de H3, agonistes de TNF, antagonistes de CRF, antagonistes de CRF BP, agonistes d'urocortine, agonistes β3, agonistes de MSH (mélanostimuline), agonistes de CCK, inhibiteurs de recaptage de sérotonine, composés mixtes sérotoninergiques et noradrénergiques, agonistes de 5HT, agonistes de bombésine, antagonistes de galanine, hormones de croissance, facteurs de libération d'hormone de croissance, agonistes de TRH, modulateurs de protéine de découplage 2 ou 3, agonistes de leptine, agonistes de DA (bromocriptine, doprexine), inhibiteurs de lipase/amylase, modulateurs de PPAR, modulateurs de RXR ou agonistes de TR-β ou amphétamines.

9. Utilisation des composés de formule I selon la revendication 8 pour la fabrication d'un médicament pour le traitement et/ou la prévention de troubles du métabolisme des acides gras et de troubles d'utilisation du glucose.

10. Utilisation des composés de formule I selon la revendication 8 pour produire un médicament pour le traitement et/ou la prévention de dyslipidémies et des séquelles de celles-ci.

11. Utilisation des composés de formule I selon la revendication 8 pour la fabrication d'un médicament pour le traitement et/ou la prévention de pathologies associées au syndrome métabolique et de troubles dans lesquels l'insulinorésistance est impliquée, ou pour le traitement et/ou la prévention du diabète sucré et des séquelles associées à celui-ci.

12. Utilisation des composés de formule I selon la revendication 8 pour produire un médicament pour le traitement et/ou la prévention de pathologies associées à un taux de HDL réduit.

13. Utilisation des composés de formule I selon la revendication 8 pour produire un médicament pour le traitement et/ou la prévention de troubles athérosclotiques.

14. Utilisation des composés de formule I selon la revendication 8 en combinaison avec au moins un principe actif supplémentaire pour produire un médicament pour le traitement et/ou la prévention de troubles dans lesquels l'insulinorésistance est impliquée.

15. Procédé pour produire un médicament comprenant un ou plusieurs des composés de formule I selon la revendication 8, qui comprend le mélange de ces derniers avec un véhicule pharmaceutiquement acceptable et la conversion de ce mélange en une forme adaptée pour administration.
